# EUROPEAN PATENT APPLICATION

(11) **EP 1 647 595 A1**
(43) Date of publication of application: **19.04.2006**
(21) Application number: 04077855.7
(22) Date of filing: 15.10.2004
(51) Int. Cl.: C12N 15/11

(54) **Nucleic acids against viruses, in particular HIV**

(71) Applicant: Academisch Medisch Centrum bij de Universiteit van Amsterdam, 1105 AZ Amsterdam (NL)
(72) Inventor: Berkhout, Benjamin, 1412 GH Naarden (NL); Baldwin, Christopher Eric, 1098 GP Amsterdam (NL)
(74) Representative: Winckels, Johannes Hubertus F.

(57) **Abstract**

The invention provides a nucleic acid molecule comprising a sequence which is complementary to a mutant of a genomic nucleic acid sequence of an organism capable of mutating its genome. Said genomic nucleic acid sequence preferably comprises a conserved, essential region. Furthermore, said genomic nucleic acid sequence preferably comprises a coding sequence and a DNA/RNA regulatory sequence. A pharmaceutical composition and a gene delivery vehicle comprising a nucleic acid molecule of the invention is also provided, as well as a method for counteracting an organism comprising providing said organism with said nucleic acid molecule. Said organism is preferably additionally provided with a nucleic acid sequence complementary to a genomic nucleic acid sequence of said organism.

## Description

The invention relates to biology. More specifically, the invention relates to counteracting an organism.

Infectious diseases caused by (micro)organisms such as for instance bacteria and/or viruses are a common phenomenon worldwide. A problem involved with counteracting (pathogenic) organisms is their capability of developing resistance against a certain treatment. Evolvement of resistant organisms often involves alterations in the genome of such organisms. Escape mutants evolve during prolonged periods of treatment. For instance, bacteria become resistant to antibiotic treatment and escape mutant viruses evolve during antiviral therapy. In view of this, therapy directed against pathogenic organisms capable of mutating their genomes often involves a combination of approaches. Despite of such combination therapy, escape mutants are still a major concern during treatment of a variety of infectious diseases.

It is an object of the present invention to provide additional means and methods for counteracting an organism capable of mutating its genome.

The invention provides a nucleic acid molecule comprising a sequence which is complementary to a mutant of a genomic nucleic acid sequence of an organism capable of mutating its genome. The invention furthermore provides a nucleic acid molecule or a binding molecule capable of specifically binding a mutant of a genomic nucleic acid sequence of an organism capable of mutating its genome, or an expression product thereof. The invention provides a different approach for avoiding and/or counteracting the occurrence of escape mutants during a certain treatment of (micro)organisms. In the art, attempts to avoid escape mutants involve a combination of several drugs/treatments which are directed towards different parts and/or different functions of a certain (micro)organism. For instance, different genomic sequences are targeted. The present invention provides the insight that a treatment is improved if a nucleic acid or any kind of binding molecule is administered which is capable of specifically binding (a gene product of) an escape mutant. Hence, escape mutants evolving during current therapies and resulting in loss of protection, are now counteracted by a nucleic acid molecule and/or binding molecule of the present invention. Therefore, protection is now maintained.

An organism is capable of mutating its genome if at least one nucleic acid sequence of its genome alters over time. This alteration can already be present at the time of the infection, or one day after infection, or after some weeks/months or it can even take years (for example 10 years) after infection before such alterations are present. The frequency and the time point at which the mutations/alteration occur depend for example on the virus type and/or the type of medicine(s) and/or the way in which the medicines are used. Preferably, said at least one nucleic acid sequence alters during treatment as a result of selective pressure. Escape mutants are favoured during a treatment targeting a wild type organism because such treatment does not affect escape mutants. If a mutant evolves that is capable of performing all, if not most, essential functions of said organism, such mutant will be capable of outgrowing while a wild type organism is suppressed by said treatment. A nucleic acid molecule or binding molecule of the present invention is preferably directed towards such escape mutant. Use of said nucleic acid molecule during treatment allows for avoiding and/or counteracting the appearance of said escape mutant.

Preferably, the invention provides a therapy based on at least one nucleic acid molecule or binding molecule that comprises a sequence which is complementary to a mutant of a genomic nucleic acid sequence of an organism capable of mutating its genome, which therapy (compared to other therapies) prolongs the time period before an escape emerges. Moreover, the faster an organism is capable of mutating its genome, the more important it is to avoid and/or counteract the occurrence of escape mutants with a nucleic acid molecule or binding molecule of the invention.

Said organism preferably comprises a pathogenic organism. In one embodiment said organism comprises a microorganism. Preferably, said organism comprises a virus or a bacterium. In one embodiment said organism comprises a DNA or RNA virus, since viruses are generally well capable of developing escape mutants. More preferably said organism comprises an RNA virus, most preferably a retrovirus. Antiviral treatment often involves occurrence of escape mutants caused by the error-prone nature of the reverse transcriptase enzyme. In one preferred embodiment said organism comprises HIV, Hepatitis B, Hepatitis C, SARS, Ebola, Influenza, West Nile and/or polio virus. Escape mutants of these pathogens appear for example in case of prolonged culturing with an antiviral agent and upon prolonged therapy. A nucleic acid molecule or binding molecule of the invention is however suitable for inhibition of many other kinds of organisms capable of mutating their genome. By inhibition of an organism is meant herein that at least one function of said organism is significantly impaired. Preferably at least one essential function of said organism is significantly impaired. In a most preferred embodiment said organism has lost its capability of replicating and/or spreading.

A nucleic acid molecule comprises a sequence which is complementary to a mutant of a genomic nucleic acid sequence if said nucleic acid molecule comprises an anti-sense strand complementary to the sense strand of a mutant of said genomic nucleic acid sequence. Said mutant comprises at least one mutation as compared to the wild type genomic sequence. For instance, said mutant comprises at least one nucleotide alteration, substitution, deletion or addition. Said mutant has preferably retained all essential functions of said organism. A nucleic acid molecule of the invention preferably comprises DNA and/or RNA. More preferably, a nucleic acid molecule of the invention comprises double stranded RNA in order to use RNA interference to degrade RNA of an undesired organism, as explained below. In other embodiments a nucleic acid molecule of the invention comprises other kinds of nucleic acid structures such as for instance a DNA/RNA helix, peptide nucleic acid (PNA), locked nucleic acid (LNA) and/or a ribozyme.

Said genomic nucleic acid sequence for instance comprises an RNA sequence, such as an HIV genomic sequence. In that case, a mutant of said genomic RNA sequence comprises said RNA sequence with at least one mutation. A nucleic acid molecule of the present invention comprises a strand which is complementary to said mutant RNA sequence (an anti-sense strand of said mutant RNA sequence).

In case a mutant of a genomic nucleic acid sequence comprises a mutated DNA sequence, a nucleic acid molecule of the invention preferably comprises a nucleic acid sequence which is complementary to the sense strand of said mutated DNA sequence. A nucleic acid molecule which is complementary to the sense strand of a mutated DNA sequence is capable of targeting said sense strand, as well as targeting an mRNA derived from said mutated DNA sequence.

A nucleic acid molecule of the invention preferably comprises double stranded nucleic acid, more preferably double stranded RNA, comprising both the sense strand and the anti-sense strand of a mutant nucleic acid sequence.

A nucleic acid molecule and/or a binding molecule of the present invention is particularly suitable for counteracting an escape mutant of an undesired organism. For instance, a (risk of) disease involving the presence of a pathogenic microorganism is counteracted by administration of a nucleic acid molecule and/or a binding molecule of the present invention. Preferably, treatment of a (pathogenic) organism involves counteracting both a wild type microorganism and at least one escape mutant thereof. An escape mutant is defined as a mutant of an organism which is capable of proliferating during a treatment which counteracts other strains of said organism. A therapy counteracting a wild type microorganism is preferably combined with administration of a nucleic acid molecule or a binding molecule of the present invention. This way, a wild type microorganism and at least one escape mutant thereof are counteracted, allowing long term protection. In a preferred embodiment a therapy targeting a certain wild type nucleic acid sequence of a microorganism is combined with administration of a nucleic acid molecule or binding molecule of the present invention capable of specifically binding a mutant of said wild type nucleic acid sequence. In this embodiment the same nucleic acid sequence of a (micro)organism, or gene product thereof, remains targeted by a combined treatment of the present invention, even when alterations in said sequence occur. In a more preferred embodiment a treatment targeting at least two different wild type nucleic acid sequences of an organism is combined with administration of nucleic acid molecules or binding molecules of the invention capable of specifically binding a mutant of said at least two different wild type nucleic acid sequences.

The sequence of a mutant of a genomic nucleic acid sequence of an organism is determined in various ways. Various kinds of mutations are predictable. A mutant organism for instance needs to remain its capability of performing essential functions. Therefore, mutations in an essential region are preferably avoided. If a treatment is specifically directed to such essential region, a so-called "silent mutation" will preferably arise in order to circumvent said treatment. A "silent mutation" is a mutation that does not, or to a low extent, interfere with any (multiple) function of a nucleic acid sequence. For instance, a coding region is preferably mutated such that the resulting codons still encode the same amino acid sequence. This is performed by a substitution in the first, second or third nucleotide of a codon such that the resulting codon encodes the same amino acid residue. Alternatively, a nucleotide substitution resulting in a conservative amino acid substitution is allowable. A conservative amino acid substitution is defined as a substitution of one amino acid with another with generally similar properties (size, hydrophobicity, etc), such that the overall functioning is not seriously affected. Mutations, such as nucleotide substitutions, resulting in conservative amino substitutions are readily calculated by a skilled person.

An escape mutant is also predicted by computing overall structure of nucleic acid variants. For instance, a mutation outside a certain essential region sometimes results in a significantly altered structure of the resulting mutant nucleic acid sequence. Such structure alterations are computed, for instance using a modeling program known in the art, and a nucleic acid molecule and/or binding molecule of the invention is generated targeting a part of the resulting nucleic acid sequence which has become available and is capable of being exposed to drugs, preferably as a result of said structure alteration.

Alternatively, the sequence of a mutant of a genomic nucleic acid sequence of an organism is determined by culturing said organism during a prolonged period in the presence of a certain (candidate) drug and determining what kind of mutations arise. Subsequently, a nucleic acid molecule or a binding molecule of the invention capable of specifically binding at least one of such mutant nucleic acids, or gene product thereof, is generated. Preferably a nucleic acid molecule of the invention is generated that comprises a sequence which is complementary to at least one of said mutants. More preferably, nucleic acid molecules of the invention are generated comprising sequences complementary to most, or all, of said mutants. In order to counteract said organism *in* vivo, said nucleic acid molecule (s) and/or binding molecule(s) of the invention is/are preferably administered together with said (candidate) drug.

If a treatment involves counteracting a function of a certain genomic nucleic acid sequence, at least one kind of escape mutant capable of evolving is predicted and/or determined experimentally. Subsequently, a nucleic acid molecule or a binding molecule of the invention capable of specifically binding at least one of such mutants is generated. Preferably nucleic acid molecules and/or binding molecules of the invention are generated capable of specifically binding most, if not all, predicted and/or determined mutant nucleic acid sequences, or gene products thereof. Preferably a nucleic acid molecule of the invention is generated that comprises a sequence which is complementary to at least one of said mutants. In one embodiment said nucleic acid molecule and/or binding molecule is administered to a patient suffering from, or at risk of suffering from, infection by said organism. Said nucleic acid molecule and/or binding molecule is preferably administered together with a treatment against a wild type form of said organism. In that case, development of escape mutants is counteracted and/or avoided because such escape mutants are immediately targeted upon appearance. Alternatively, said nucleic acid molecule and/or binding molecule is administered once said escape mutant has evolved.

In order to efficiently counteract an organism, an essential region of said organism's genome, or a gene product thereof, is preferably targeted. If a non-essential region is targeted, escape mutants readily arise because mutations in a non-essential region mostly do not inhibit proliferation. An organism is often capable of deleting a large part of a non-essential region in order to escape from treatment, without impairing proliferation. Hence, an essential region is preferably targeted in order to significantly limit the organism's possibilities of forming escape mutants. Yet, escape mutants are reported to evolve, especially when highly mutating organisms such as RNA viruses are targeted. Therefore, a nucleic acid molecule or binding molecule of the present invention is preferably capable of specifically binding a mutant of an essential nucleic acid region of an organism, or an expression product thereof. More preferably a nucleic acid molecule of the invention is complementary to a mutant of an essential nucleic acid region of an organism. Therapy targeting at least one essential region of a wild type organism, together with a nucleic acid molecule and/or binding molecule of the invention targeting a mutant of said at least one essential region, is capable of long time suppression of an organism.

In order to be capable of counteracting various strains of a certain organism, a nucleic acid molecule or binding molecule of the invention is preferably capable of specifically binding at least part of a conserved region. A conserved region is defined as a region which is at least 90% identical in at least 50% of the (known) genomes of various strains of the same kind of organism. Said conserved region is preferably at least 95%, more preferably at least 98%, most preferably 100% identical in at least 50% of the known genomes of various strains of the same kind of organism. Said conserved region is preferably at least 95%, more preferably at least 98%, most preferably 100% identical in at least 75% of the known genomes of various strains of the same kind of organism. In one aspect a nucleic acid molecule of the invention is provided which is complementary to a mutant of a genomic nucleic acid sequence of an organism, wherein said genomic nucleic acid sequence comprises at least part of a conserved region. Said part of a conserved region preferably comprises at least 5, more preferably at least 10, more preferably at least 15, more preferably at least 17, more preferably at least 18, more preferably at least 19, more preferably at least 20, more preferably at least 22 nucleotides, most preferably at least 23 nucleotides of said conserved region. The higher the amount of nucleotides of said part, the higher the chance that a nucleic acid molecule of the invention specifically binds a mutant of any given strain of said organism.

In the art various methods are known for counteracting an organism by targeting a genomic nucleic acid sequence or a gene product thereof. For instance, antisense DNA oligonucleotides are used since the 1980s in order to silence gene expression. Moreover, ribozymes capable of catalyzing cleavage of target RNAs are used. A preferred method for gene silencing is RNA interference (RNAi). RNA interference is a sequence-specific RNA degradation process in the cytoplasm of eukaryotic cells that is induced by double stranded RNA. This RNA-silencing mechanism, which was first described in *Caenorhabditis elegans* and *Drosophila melanogaster* has many similarities with post-transcriptional gene silencing in plants. RNAi and related RNA silencing mechanisms are believed to act as a natural defence against incoming viruses and the expression of transposable elements. Besides the antiviral function of RNAi there is evidence that RNAi plays an important role in regulating cellular gene expression. These features have characterized RNAi both as an ancient and fundamentally important mechanism in eukaryotic cell biology.

It has been shown that the process is initiated via so-called small interfering RNAs (siRNAs) of approximately 21-23 base pairs (bp), which are cleaved from double-stranded precursor RNAs by the RnaseIII-like enzyme DICER. These siRNAs associate with various proteins to form the RNA-induced silencing complex (RISC), harbouring nuclease and helicase activity. The antisense strand of the siRNA guides the RISC to the complementary target RNA, and the nuclease component cleaves the target RNA in a sequence-specific manner. Hence, double stranded RNA is capable of inducing degradation of the homologous single stranded RNA in a host cell.

Synthetic siRNAs of 21 bp are shown to efficiently induce RNAi-mediated gene silencing when transfected into mammalian cells. In addition, RNAi are induced in mammalian cells by intracellularly expressed short hairpin RNAs (shRNAs), preferably with a length of 19 bp, with a small loop.

Viruses are both inducers and targets of RNA silencing in plants. Similarly, RNAi has been shown to be induced by virus replication in animals. The antiviral capacity of RNA silencing has been used as a tool to generate virus resistance in plants. RNA-mediated virus resistance was obtained by expression of untranslatable viral coat-protein RNAs in transgenic plants. Expression of hairpin RNAs corresponding to viral sequences induced virus resistance in almost 100% of the transgenic plants. In analogy with RNA-mediated virus resistance in plants, RNAi technology is currently being used to inhibit viral replication in animal cells. Promising results have been obtained with RNAi against several animal viruses both *in in vitro* and in *vivo* settings. For instance, Das et al have demonstrated that inhibition of HIV-1 replication through RNAi is possible in stably transduced cells. However, HIV-1 escape mutants that were no longer inhibited appeared after several weeks of culture (Das et al. Journal of Virology, Vol 78, No. 5 (2004) pages 2601-2605). Moreover, Gitlin et al have shown that pre-treatment of human and mouse cells with double-stranded short interfering RNAs to the poliovirus genome markedly reduces the titre of virus progeny and promotes clearance of the virus from most of the infected cells. Protection is the result of direct targeting of the viral genome by siRNA. Also in this study escape variants appeared (Gitlin et al. Nature, Vol 418 (2002) pages 430-434).

The present invention provides a way of improving RNAi based treatment of an infection. With a method of the invention a disease involving the presence of an organism is prevented and/or treated. A second generation of interfering RNAs is provided capable of counteracting escape mutants. Hence, long term suppression of a mutating organism has become possible. In one preferred embodiment a nucleic acid molecule of the present invention therefore comprises small interfering RNA.

A host cell is for instance provided with double stranded RNA by transfection of double stranded RNA molecules such as siRNA. siRNAs are for instance chemically or enzymatically synthesized. If long-term suppression of an organism is required, it is preferred to provide a cell with an expression cassette so that a double stranded RNA is produced by the host cell. Intracellular synthesis of an siRNA is for instance achieved by expression of separate sense and antisense RNA fragments, which together form a dsRNA. Preferably however, a short hairpin RNA (shRNA) is expressed. A short hairpin RNA comprises at least one base paired stem (also called a base paired duplex) comprising the sense and antisense strand of a siRNA, preferably with a length of between 15-40 nucleotides, more preferably with a length of between 15-30 nucleotides, more preferably with a length of between 19 and 25 nucleotides, most preferably with a length of between 21-23 nucleotides. The sense and antisense strand of an shRNA are linked with a small loop.

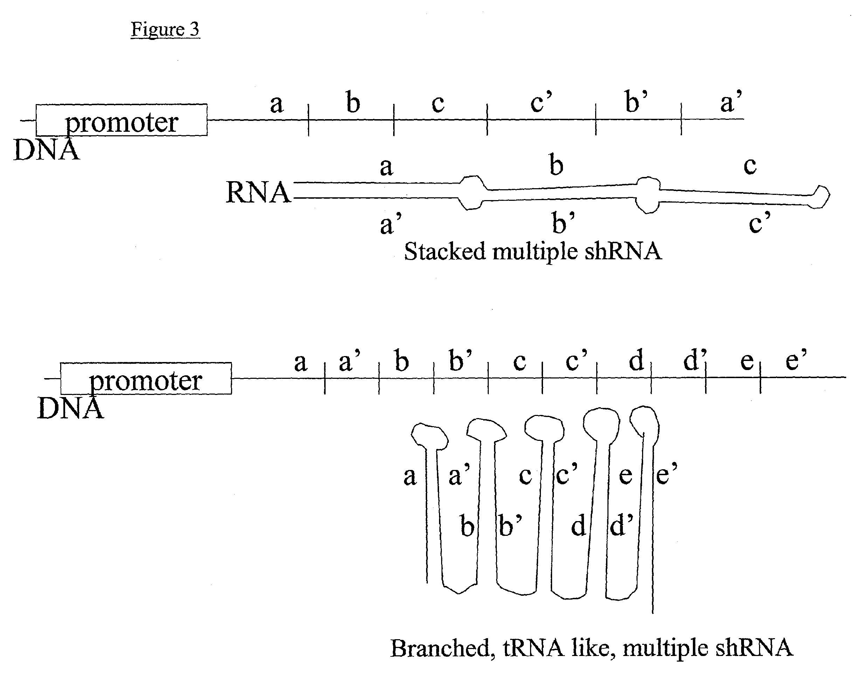

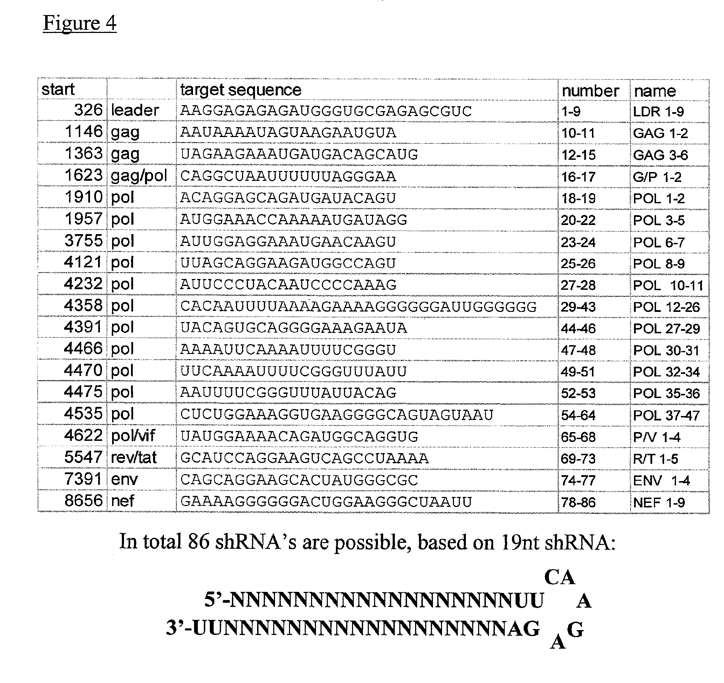

If a single short hairpin is used, the sense and antisense strands are joined through a single-stranded loop of several nucleotides. In one embodiment, multiple shRNA expression cassettes are inserted into a single vector. The units are clustered (parallel or antiparallel orientation) or inserted at different positions of the vector. In a preferred embodiment multiple short hairpin RNAs are combined in a single expression construct. For instance, a stacked multiple shRNA structure is used wherein the dsRNA sequences are separated by bulges, or a branched, tRNA-like multiple shRNA structure is used wherein each branch is a separate shRNA. The efficiency of multiple shRNA constructs is preferably optimized by inclusion of signals capable of determining the intracellular location and stability of the RNA transcript. A multi-shRNA transcript is preferably made from a polymerase II or a polymerase III promoter. Examples of multi-shRNA constructs are shown in figure 3 and in Anderson et al, Oligonucleotides 13:303-312(2003).

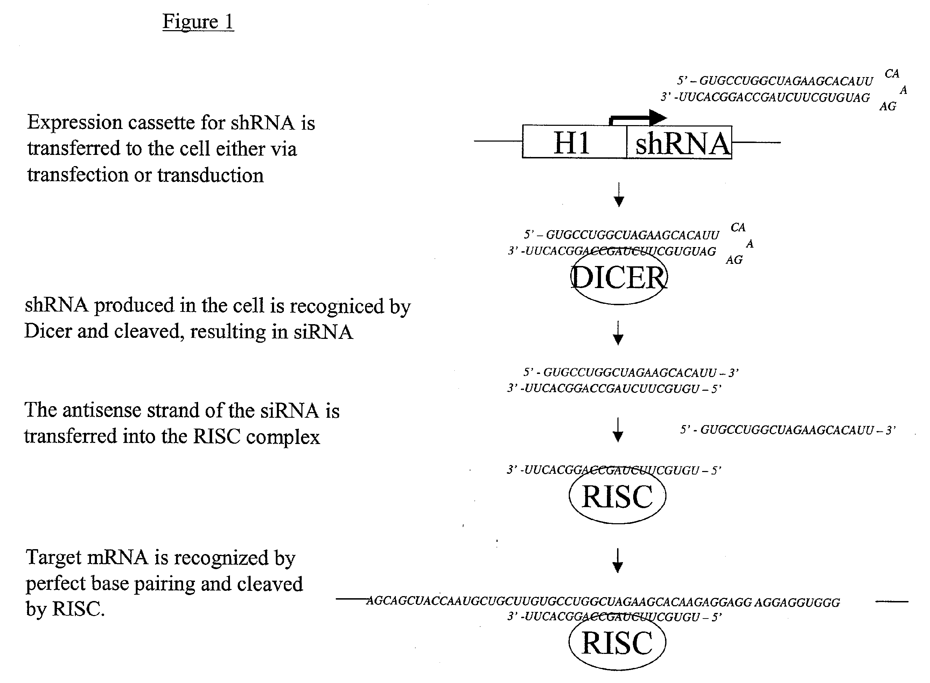

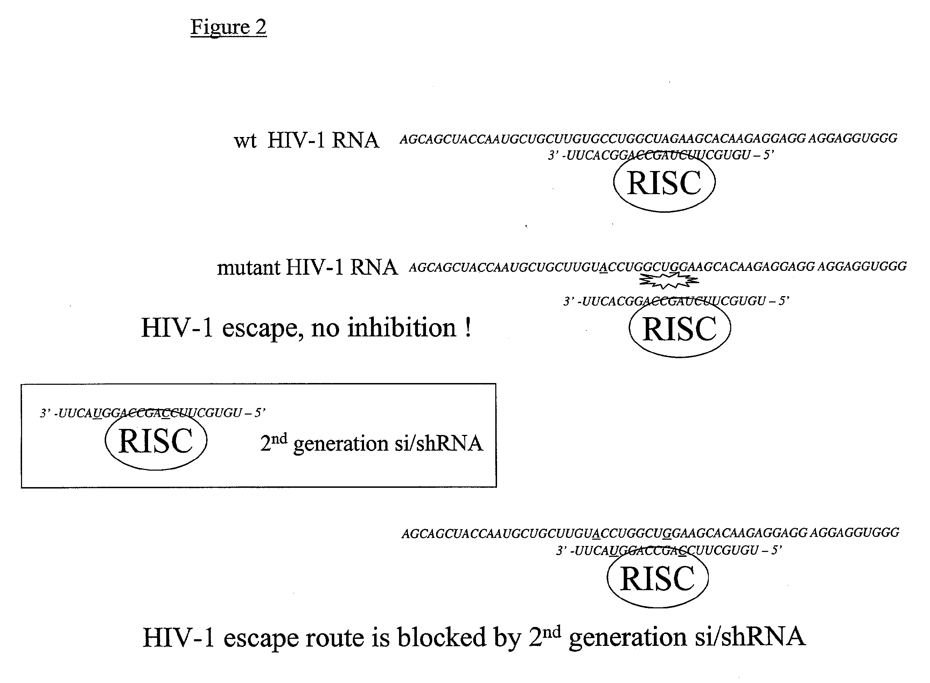

A short hairpin RNA is recognized by the DICER enzyme. Processing of a functional shRNA by DICER yields a siRNA of which the antisense strand is transferred to RISC, finally resulting in RNAi. Figure 1 provides a schematic overview of RNAi interference with shRNA. Activation of the RNAi machinery by intracellular expression, preferably expression of a shRNA, has the advantage over transfection of synthetic siRNA that there is a constitutive transcription ensuring a basal level of RNAi activity. One aspect of the invention therefore provides a nucleic acid molecule of the invention comprising short hairpin RNA. In one embodiment said shRNA comprises a single short hairpin. In another embodiment said shRNA comprises a multiple short hairpin structure. A single shRNA of the invention preferably comprises a loop with a length of about 2 to 15 nucleotides. More preferably said loop has a length of about 3 to 10 nucleotides, most preferably about 5 to 8 nucleotides. In one embodiment said loop comprises hairpin-stabilizing tetraloop sequences. The basepaired duplex of an extended shRNA of the invention is furthermore preferably destabilized. This is for instance performed by inclusion of weak G-U base pairs and/or mispairs, and/or by destabilizing bulge or internal loop elements by modification of the sense strand.

Whereas the standard expression strategy for a shRNA transcript is the use of a Polymerase III (Pol III) promoter (e.g. H1 or U6), the expression of a longer transcript encoding multiple shRNAs is preferably optimized. This for instance includes the use of a Polymerase II (Pol II) promoter or the T7 expression system for cytoplasmic RNA expression. A promoter used in a short hairpin construct of the present invention is for instance constitutively expressed, either in all cell types/tissues or in a cell/tissue-specific manner. Preferably however said promoter is inducibly active so that the amount of expression of double standed RNA is regulated. A variety of viral vector systems is suitable for providing a host cell with a nucleic acid molecule of the invention. Preferably, a lentiviral and/or retroviral vector is used.

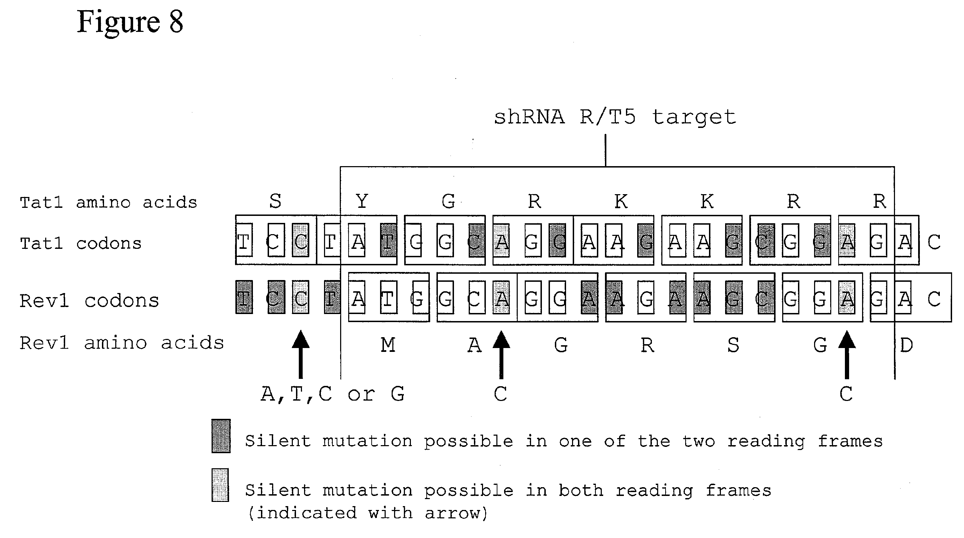

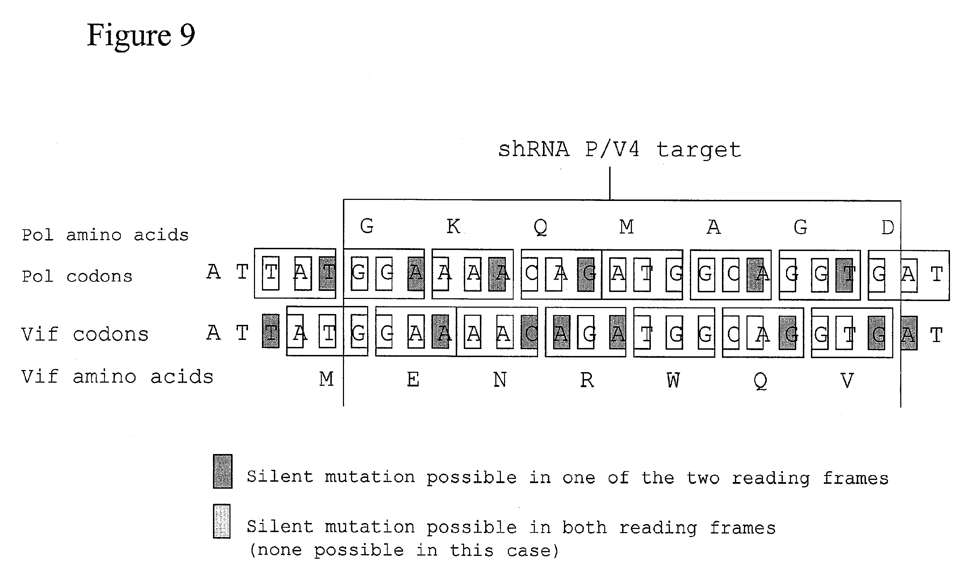

A method of the invention preferably involves targeting of an essential, conserved region so that an organism is limited in its capacity of circumventing treatment via escape mutants. In a preferred embodiment at least one genomic region of an organism is targeted that executes multiple functions. For instance, a region with an overlapping reading frame or even a triple overlap is targeted. An example of such region is the Tat-Rev-Env region in HIV. In one embodiment a region comprising both a coding sequence and a regulatory sequence is targeted. Such region for instance comprises a sequence encoding a protein and an RNA/DNA replication signal such as a splicing signal or a sequence motif that controls RNA packaging or reverse transcription. If such regions are targeted, escape mutants are more difficult to arise because all functions need to be retained. A mutation which maintains one function may impair another function to such extent that the mutant is not viable. In view of these restrictions, the number of possible escape mutants is limited. Hence, if each possible escape mutant is to be targeted by a nucleic acid molecule or binding molecule of the invention, a limited number of nucleic acid molecules or binding molecules of the invention is needed. Mutations that would result in an escape mutant retaining all essential functions are readily computed or determined experimentally, as for instance illustrated by example 2 and figures 8 and 9. One aspect of the invention therefore provides a nucleic acid molecule of the invention wherein said genomic nucleic acid sequence of said organism comprises at least one coding sequence and/or at least one DNA/RNA regulatory sequence.
Another aspect provides a nucleic acid molecule of the invention comprising a sequence which is complementary to a mutant comprising a coding sequence of an organism wherein a nucleotide of a codon is substituted such that the resulting codon encodes the same amino acid residue. As illustrated by example 2 and figure 8, possible escape mutants are readily computed.

In another embodiment a nucleic acid molecule of the invention is provided, wherein said mutant comprises a genomic nucleic acid sequence of said organism with at least one mutation resulting in a significantly altered structure of said genomic nucleic acid sequence. The present inventors have identified a novel resistance mechanism through the formation of a stable nucleic acid structure by at least one mutation in or near the targeted sequence. Such mutants comprising an altered nucleic acid structure are computed using modelling programs known in the art such as computer-assisted RNA structure prediction programs. Alternatively, they are detected using long term cultures in the presence of a (candidate) drug, such as for instance an antiviral. A nucleic acid molecule or binding molecule of the present invention is capable of binding such altered nucleic acid structure.

A nucleic acid molecule of the invention preferably comprises an inducible repressor and/or activator. In that case the amount of expression, and therefore the degree of treatment, is regulated. For instance, nucleic acid molecules complementary to all possible escape mutants are administrated. Expression is repressed until a certain escape mutant has evolved. Upon detection of such escape mutant, expression is enhanced by deactivating a repressor and/or activating an activator. In another embodiment, expression of a nucleic acid molecule of the invention is activated during treatment in order to prevent viral escape. In one embodiment expression of a nucleic acid molecule of the invention is induced or repressed depending on possible side effects of a treatment and/or general health state of a patient.

A nucleic acid molecule and/or a binding molecule of the present invention is suitable for treating or preventing a disease involving the presence of an organism. One aspect of the invention therefore provides a nucleic acid molecule and/or binding molecule of the invention for use as a medicament. A nucleic acid molecule and/or binding molecule of the invention is particularly suitable for the preparation of a medicament or a vaccine against a disease involving an organism capable of mutating its genome. Said organism preferably comprises HIV, Hepatitis B, Hepatitis C, SARS, Ebola, Influenza, West Nile and/or polio virus because these organisms have a high mutation rate.

A pharmaceutical composition comprising a nucleic acid molecule and/or binding molecule of the invention and a suitable diluent or carrier is also herewith provided. In one embodiment said pharmaceutical composition comprises a vaccine.

Treatment of a (pathogenic) organism is particularly effective if a wild type form of said organism is counteracted as well. One aspect of the invention therefore provides a combination therapy wherein at least one compound capable of counteracting a wild type organism is combined with a nucleic acid molecule and/or binding molecule of the present invention. Many compounds capable of counteracting a wild type organism are known in the art. In this embodiment at least one of these compounds is combined with a nucleic acid molecule or a binding molecule of the invention. Said compound capable of counteracting a wild type organism preferably targets at least one wild type nucleic acid sequence of said organism. Said compound preferably comprises double stranded RNA, such as siRNA and/or shRNA, in order to use RNA interference. A compound capable of targeting a wild type sequence is preferably combined with a nucleic acid molecule or binding molecule of the invention capable of specifically binding a mutant of the same wild type sequence. In one embodiment a second generation shRNA of the invention is combined with a primary shRNA capable of targeting a wild type sequence. Alternatively, another kind of drug therapy is combined with a nucleic acid molecule or binding molecule of the invention. For instance, a currently used drug such as for instance an antiviral is combined with a nucleic acid molecule or binding molecule of the invention capable of specifically binding an escape mutant which is resistant to said drug. In this way variants of an organism that are resistant to said drug are targeted by said nucleic acid molecule and/or binding molecule of the invention while variants resistant to said nucleic acid molecule and/or binding molecule of the invention are targeted by said drug. Various drug-resistance mutations are well known in the art. For instance, HIV drug-resistance mutations are included in the listing of the International AIDS Society (IAS) to help physicians classify the level of drugs-resistance. A nucleic acid molecule or binding molecule of the invention is preferably capable of specifically binding at least one of said drug-resistance mutants.

In another embodiment frequently used CTL-escape or antibody escape routes of mutating organisms are blocked by a nucleic acid molecule and/or binding molecule of the invention anticipating such changes in the organism's genome.

A compound capable of counteracting a wild type organism is preferably combined with a nucleic acid molecule and/or binding molecule of the invention in one pharmaceutical composition. In one embodiment said compound capable of counteracting a wild type organism and a nucleic acid molecule and/or binding molecule of the invention are administered at the same time. Alternatively, a compound capable of counteracting a wild type organism and a nucleic acid molecule and/or binding molecule of the invention are administered at different time points. For instance, a nucleic acid molecule and/or binding molecule of the invention is administered once an escape mutant has evolved.

One aspect of the invention therefore provides a pharmaceutical composition comprising a nucleic acid molecule comprising a sequence which is complementary to a mutant of a genomic nucleic acid sequence of an organism capable of mutating its genome, and a nucleic acid molecule comprising a sequence which is complementary to a genomic nucleic acid sequence of said organism. Preferably, said nucleic acid molecule of the invention and said second nucleic acid molecule complementary to a genomic nucleic acid sequence are both complementary to (a mutant of) the same nucleic acid region of said genome. A pharmaceutical composition of the invention comprising a nucleic acid molecule of the invention, or binding molecule of the invention, and another kind of drug capable of at least in part counteracting said disease is also herewith provided. Said other kind of drug for instance comprises a known antiviral drug.

Efficiency of treatment is enhanced when various nucleic acid sequences of an organism, or gene products thereof, are targeted because a mutant comprising a mutation in one nucleic acid sequence will still be targeted by a compound capable of targeting a second nucleic acid sequence of said organism. Escape mutants comprising a mutation in various nucleic acid sequences are more difficult to evolve as compared to escape mutants comprising a mutation in just one nucleic acid sequence. If escape mutants comprising a mutation in various nucleic acid sequences evolve, they are treated with a nucleic acid molecule or binding molecule of the invention. Hence, a preferred embodiment comprises a pharmaceutical composition comprising nucleic acid sequences which are complementary to mutants of at least two genomic nucleic acid sequences of said organism. More preferably, said pharmaceutical composition comprises nucleic acid sequences which are complementary to mutants of at least four genomic nucleic acid sequences of said organism. Most preferably, a pharmaceutical composition is provided comprising nucleic acid sequences which are complementary to mutants of at least six genomic nucleic acid sequences of said organism. The higher the number of genomic nucleic acid sequences that, once they are mutated, are targeted by nucleic acid molecules and/or binding molecules of the invention, the higher the percentage of inhibition.

A nucleic acid sequence of the invention is administered in various ways known in the art. For instance, siRNAs are orally administered or injected. Said siRNAs are preferably administered with a suitable diluent or carrier, such as for instance a physiologic salt solution. Preferably, cells are transduced with an expression cassette, preferably a shRNA construct. A gene delivery vehicle such as a viral vector system, preferably a lentiviral and/or retroviral vector is particularly suitable. Methods of generating and using a gene delivery vehicle are well known in the art. Preferably vectors and protocols according to (Dull et al., Journal of Virology 72, 8463-8471, 1998 and/or Seppen et al., Journal of Hepatology, Apr;36(4); 459-65, 2002) are used. A gene delivery vehicle comprising a nucleic acid sequence of the invention is therefore also herewith provided. Said gene delivery vehicle is suitable for ex *vivo* and in *vivo* gene therapy.

A method of the invention is particularly suitable for counteracting an organism. Preferably said organism is counteracted in *vivo,* comprising administration of a nucleic acid molecule or binding molecule of the invention to an individual suffering from, or at risk of suffering from, infection by said organism. In vitro applications comprise counteracting contaminants in (cell) culture. The invention thus provides a method for at least in part counteracting an organism, comprising providing said organism with a nucleic acid molecule or binding molecule of the invention. Said organism is preferably additionally provided with a nucleic acid molecule comprising a sequence which is complementary to a genomic nucleic acid sequence of said organism. In that case both wild type forms and escape mutants are targeted. Targeting of multiple genomic nucleic acid sequences enhances the efficiency of a method of the invention. A preferred embodiment therefore provides a method of the invention wherein said organism is provided with nucleic acid sequences which are complementary to mutants of at least two, more preferably at least four, most preferably at least six genomic nucleic acid sequences of said organism.

A nucleic acid of the invention is suitable for interfering with a cell comprising an organism capable of mutating its genome, and/or with a cell comprising a nucleic acid sequence of an organism capable of mutating its genome. One embodiment of the invention therefore provides a method for interfering with a cell comprising an organism capable of mutating its genome and/or comprising a nucleic acid sequence of an organism capable of mutating its genome, comprising providing said cell with a nucleic acid sequence of the invention.

A method of the invention is preferably used against an organism involved with disease. Administration of a nucleic acid molecule and/or a binding molecule of the invention is capable of at least in part treating or preventing said disease during prolonged periods. The invention thus provides a method for at least in part treating or preventing a disease involved with an organism capable of mutating its genome, comprising providing an individual suffering from, or at risk of suffering from, said disease with a pharmaceutical composition of the invention. Preferably said individual is additionally provided with another kind of drug capable of at least in part counteracting said organism.

A kit of parts comprising a nucleic acid molecule and/or binding molecule of the invention and another kind of drug capable of at least in part counteracting an organism is also herewith provided.

### Detailed description

A method of the invention is particularly suitable for preventing and/or treating a virus-associated disease. Viruses such as HIV, polio, hepatitis, SARS, Ebola, Influenza and/or West Nile virus are capable of escaping antiviral therapy because of their high mutation rate.
In view of this, current therapy directed against such mutating viruses often involves a combination of approaches. For instance, current therapy against HIV, sometimes called HAART (Highly Active Anti-Retroviral Therapy), often involves a combination of a nucleoside/tide reverse transcriptase inhibitor with a protease inhibitor and/or a non-nucleoside reverse transcriptase inhibitor.

In one embodiment a method of the invention is used in order to counteract HIV. In that case mature CD4+ T cells and/or CD34+ bone marrow precursor cells are preferably provided with a nucleic acid molecule or a binding molecule of the present invention. Preferably, mature CD4+ T cells and/or CD34+ bone marrow precursor cells are provided with a shRNA of the present invention. A variety of viral vector systems is suitable for providing a host cell with a nucleic acid molecule of the invention. Preferably, a lentiviral and/or retroviral vector is used. In one embodiment an inducible promoter is used. For instance, during treatment of HIV the Tet-system for doxycycline (dox) induced expression as disclosed in WO O1/20013 is preferred, or the HIV-1 LTR promoter that is activated only in HIV-infected cells by the viral Tat protein (Berkhout et al., Cell 1989; 59:273-282). In one embodiment cytoplasmic expression using the T7 polymerase system is used for expression of shRNA, preferably multiple shRNA constructs. In one embodiment a conditionally replicating HIV-based vector system is used as disclosed in WO 01/20013. This dox-dependent virus which has been developed as a safe live-attenuated vaccine strain is also suitable for use as a therapeutic virus by insertion of a nucleic acid molecule of the present invention, such as a shRNA expression cassette of the invention.

Haematopoietic stem cells are preferably transduced with an RNAi expression cassette of the present invention. These stem cells proliferate into mature T cells that resist HIV-1 infection. The virus-resistant cell population becomes enriched in case the non-resistant cells are infected and killed, either directly by the virus or indirectly by the immune system. The preferential survival of even a minority of siRNA-expressing cells results in their dominance over time.

Besides targeting of viral RNA, an alternative way to inhibit virus replication by RNAi is to silence the expression of cellular genes that are involved in viral replication. For HIV-1, these targets include the mRNAs encoding the CD4 receptor and the CCR5 or CXCR4 co-receptors. These receptors are essential for attachment of the HIV-1 particle to the cell and subsequent viral entry. RNAi against the viral RNA does not protect the cell against viral entry. By silencing the receptors, the HIV-1 particle will be unable to attach to and to enter the cell, yielding a form of HIV-1 resistance. In *vivo* suppression of CD4 or CXCR4 is not preferred because of their immunological function. Therefore, preferably expression of the co-receptor CCR5 is silenced. An inactivating mutation in the CCR5 gene is compatible with normal life, demonstrating that it plays no essential role in human physiology.

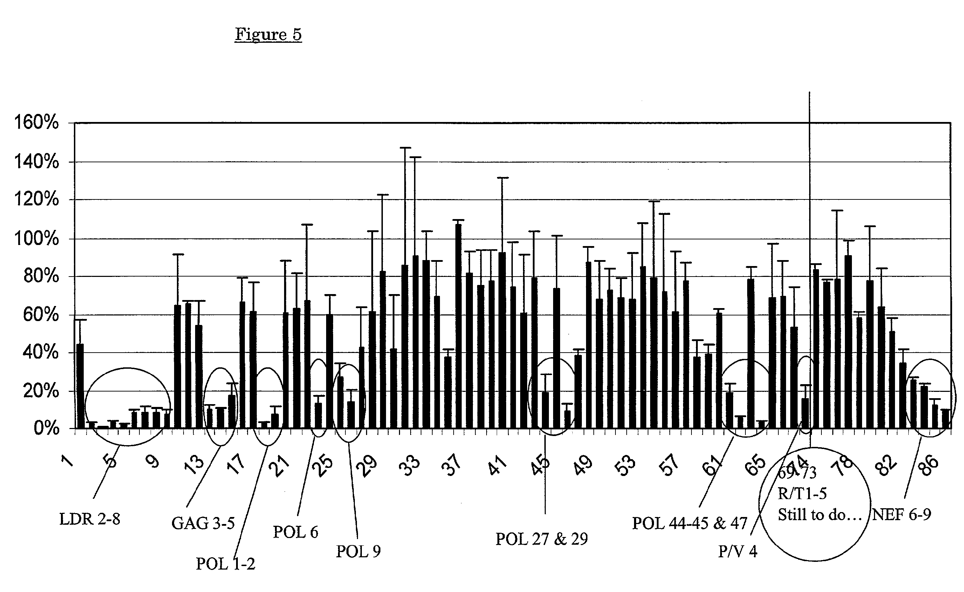

In a preferred embodiment the invention furthermore provides eleven conserved regions of HIV-1 which are especially suitable for targeting during anti HIV treatment. Although the HIV-1 genome comprises more conserved regions, it has been shown by the present inventors that the eleven conserved regions are particularly suitable for being targeted by nucleic acid silencing therapy. Silencing at least one region of said eleven conserved regions results in efficient virus inhibition. Furthermore, treatment is improved with a nucleic acid molecule or binding molecule of the invention capable of specifically binding a mutant of at least one of these eleven conserved regions. The eleven selected conserved regions are depicted in figure 5 and table 1B. In one aspect the invention therefore provides a nucleic acid molecule comprising a sequence which is complementary to a conserved region as depicted in table 1B, or to a functional part or derivative of said conserved region. Furthermore a nucleic acid molecule is provided which is complementary to a mutant of a conserved region as depicted in table 1B, or to a functional part or derivative of said mutant. In yet another embodiment the invention provides a nucleic acid molecule comprising a sequence which is complementary to the conserved R/T 1-5 region as depicted in table 1A, or to a functional part or derivative of said conserved R/T 1-5 region. Furthermore a nucleic acid molecule is provided which is complementary to a mutant of the conserved R/T 1-5 region as depicted in table 1A, or to a functional part or derivative of said mutant. The conserved R/T 1-5 region is also suitable for being targeted by nucleic acid silencing therapy, said therapy preferably comprising a nucleic acid molecule and/or binding molecule of the present invention.

A functional part of a nucleic acid sequence is defined herein as a part of said nucleic acid, at least 10 base pairs long, preferably at least 15 base pairs long, more preferably at least 19 base pairs long, comprising at least one characteristic (in kind not necessarily in amount) as said nucleic acid sequence. Targeting of a part of a conserved nucleic acid sequence of the invention by nucleic acid silencing therapy preferably results in inhibition of HIV. A derivative of a nucleic acid sequence is defined as a modified nucleic acid sequence comprising at least one characteristic (in kind, not necessarily in amount) as said nucleic acid sequence. Said derivative for instance comprises said nucleic acid sequence with at least one silent mutation. A person skilled in the art is well capable of generating an analogue of a nucleic acid sequence. Such analogue for instance comprises a peptide nucleic acid (PNA), locked nucleic acid (LNA) and/or a ribozyme. Such analogue comprises at least one characteristic (in kind, not necessarily in amount) as said nucleic acid sequence.

The invention furthermore provides a nucleic acid molecule comprising a sequence which is complementary to a sequence as depicted in table 2A, 2B or 2C, or a functional part, derivative and/or analogue thereof. Such nucleic acid molecules are capable of inhibiting HIV and are therefore suitable for therapy. If at least one of such nucleic acid molecules is used during a prolonged period, escape mutants comprising mutations within these sequences, which are still capable of performing most - if not all - essential functions, are likely to arise. These escape mutants are targeted with a nucleic acid molecule or binding molecule of the present invention which is capable of specifically binding such escape mutant. In one embodiment the invention therefore provides a nucleic acid molecule comprising a sequence which is complementary to a mutant of a sequence as depicted in table 2A, 2B and/or 2C, or a functional part, derivative and/or analogue thereof. Escape mutants retaining their essential functions are computed as discussed above, or detected using long term cultures in the presence of a nucleic acid sequence as depicted in table 2A, 2B and/or 2C.

A sequence as depicted in table 2A, 2B or 2C, or a functional part, derivative and/or analogue thereof, is capable of inhibiting HIV. Preferably, the percentage of HIV inhibition comprises at least 50 % reduction in virus titer. A preferred embodiment therefore provides a nucleic acid molecule comprising a sequence which is complementary to a sequence as depicted in table 2A or 2B, or a functional part, derivative and/or analogue thereof. These sequences are capable of inhibiting HIV with a percentage of inhibition of at least 50% and are therefore particularly suitable for therapy. Escape mutants arising as a result of selective pressure when treatment with a sequence as depicted in table 2A or 2B is used, are targeted with a nucleic acid molecule or binding molecule of the present invention which is capable of specifically binding such escape mutant. In one embodiment the invention therefore provides a nucleic acid molecule comprising a sequence which is complementary to a mutant of a sequence as depicted in table 2A or 2B, or a functional part, derivative and/or analogue thereof.

Yet another preferred embodiment provides a nucleic acid molecule comprising a sequence which is complementary to a sequence as depicted in table 2A, or a functional part, derivative and/or analogue thereof. These sequences are capable of inhibiting HIV with a percentage of inhibition of at least 80% and are therefore particularly suitable for therapy. Escape mutants arising as a result of selective pressure when treatment with a sequence as depicted in table 2A is used, are targeted with a nucleic acid molecule or binding molecule of the present invention which is capable of specifically binding such escape mutant. In one embodiment the invention therefore provides a nucleic acid molecule comprising a sequence which is complementary to a mutant of a sequence as depicted in table 2A, or a functional part, derivative and/or analogue thereof.

Yet another aspect of the invention provides a nucleic acid molecule comprising a sequence which is complementary to a sequence as depicted in table 2D, or a functional part, derivative and/or analogue thereof. A nucleic acid molecule comprising a sequence which is complementary to a mutant of a sequence as depicted in table 2D, or a functional part, derivative and/or analogue thereof, is also herewith provided. A nucleic acid sequence complementary to (a mutant of) a sequence as depicted in table 2D, or a functional part, derivative and/or analogue thereof, is also suitable for HIV therapy.

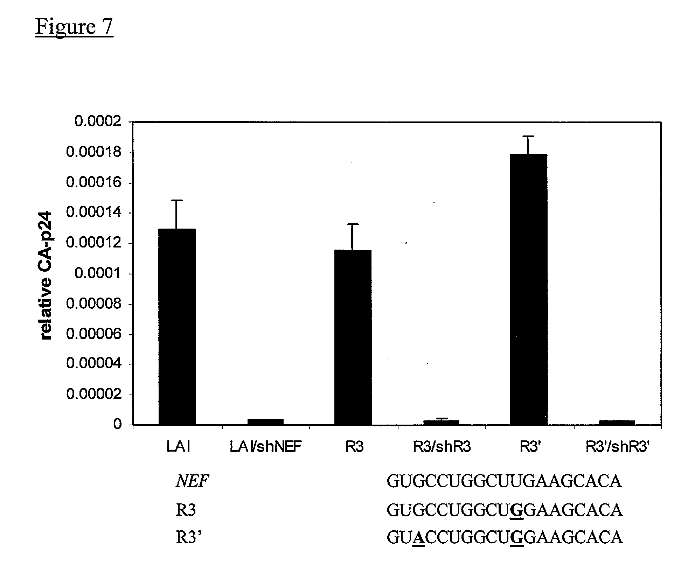

Two preferred sequences, named R3 and R3', are shown in figure 7. Administration of these sequences, preferably in the form of siRNA or shRNA, significantly improves inhibition of HIV. One aspect of the invention therefore provides a nucleic acid molecule which is complementary to the sequence GUGCCUGGCUGGAAGCACA and/or GUACCUGGCUGGAAGCACA, or a functional part, derivative and/or analogue thereof.

The invention is further illustrated by the following examples. These examples are not limiting the invention in any way.

### Examples

### Example 1

### Materials and Methods

### Conserved HIV-1 sequences

20-mer sequences covering the complete genome of the HIV-1 molecular clone pLAI were aligned with all complete HIV-1 genome sequences available, 170 at that time, from the Los Alamos database. The LAI genome size is 9229 bases, this means that in total 9211 different 20-mer sequences are possible. We defined conserved sequences as target regions that have 100% identity with the 20-mer sequence in at least 75% of all HIV-1 genomes. The conserved regions with their nucleotide position, genomic region and sequence are shown in table 1A.

### ShRNA expressing plasmids

ShRNA's expressing plasmids were constructed as follows. The pSUPER vector, a gift from T.R. Brummelkamp, the Netherlands Cancer Institute (Brummelkamp et al., 2002), that contains the H1 promoter, was linearized with BglII and HindIII. Forward and reverse strands of the oligo's, which contain the shRNA-expressing sequence targeting the conserved regions, were annealed and cloned into the vector.

The sequence targeting the conserved region was 19 nt, that means for a stretch of 23 nucleotides, 5 different shRNA's were designed. The oligo sequences were as follows:

GATCCCCNNNNNNNNNNNNNNNNNNNTTCAAGAGANNNNNNNNNNNNNNNNNNNTTTTTGGAAA

Forward primer:

sense anti-sense

GATCCCCNNNNNNNNNNNNNNNNNNNTTCAAGAGANNNNNNNNNNNNNNNNNNNTTTTTGGAAA

AGCTTTTCCAAAAANNNNNNNNNNNNNNNNNNNTCTCTTGAANNNNNNNNNNNNNNNNNNNGGG

Reverse primer

sense

anti-sense

AGCTTTTCCAAAAANNNNNNNNNNNNNNNNNNNTCTCTTGAANNNNNNNNNNNNNNNNNNNGGG

The shRNA expressing plasmids targeting the conserved regions of HIV-1 were numbered from 1 to 86 and were also numbered according to the genomic region, as is shown in table 1A.

### Co-transfectaort of shRNA constructs with pLAI

In a 96 wells format, co-transfection experiments of the shRNA-vector and pLAI were performed. Per well, 2 x 10e4 293T cells were seeded in 200 µl DMEM with 10% FCS without antibiotics. The next day, 200 ng of pLAI, 20ng of shRNA-vector and 0.63ng of pRL (Renilla Luciferase) was transfected with 0.5 µl lipofectamine-2000 in a reaction volume of 50µl according to the manufacturers instructions. Eight hours post-transfection the medium was replaced with 200 µl medium containing antibiotics, 48 hours after transfection medium samples were taken for CA-p24 ELISA and cells were lysed for Renilla measurements. CA-p24 is a measure of virus production. To correct for transfection variation, relative CA-24 values were determined by dividing the CA-p24 measurements by the Renilla values obtained. Negative controls that were included were pBS, pSUPER (without insert) and an empty control. Positive controls were shRNA's targeting the Nef gene (Das et al., 2003) and the Gag gene (Capodici et al., J.Immunol. 2002 Nov 1; 169(9): 5196-201).

Smaller scale transfections were carried out in a 24-wells format. Per well, 1-1.5 x 10e5 293T cells were seeded in 500 µl DMEM with 10% FCS without antibiotics. The next day, 500 ng of pLAI, 0-500 ng of shRNA-vector and 2.5ng of pRL (Renilla Luciferase) was transfected with 1-2 µl lipofectamine-2000 in a reaction volume of 100µl according to the manufacturers instructions. The remainder of the experiment was carried out as described above.

### Results

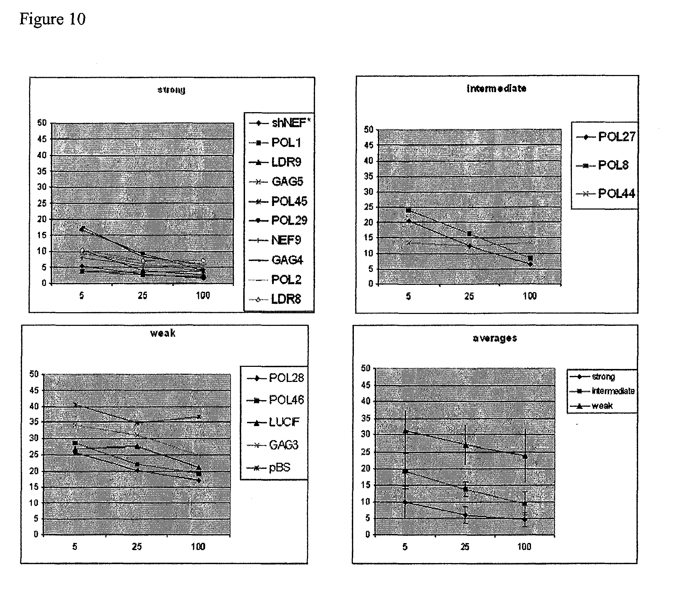

First we identified conserved regions in the HIV-1 clone pLAI. Conservedness was defined as regions in LAI having 100% identity with at least 75% of all complete HIV-1 genomes available in the Los Alamos database at that time (170). Nineteen different regions were found (table 1A). For these regions, shRNA expressing plasmids were constructed containing 19 nucleotides of the target sequences. For a conserved stretch of 25 nucleotides, this means that seven different shRNA's were made. In co-transfection experiments, all constructs were tested for their ability to inhibit HIV-1 production (figure 5, table 2). We divided the shRNA's into different groups; strong shRNA's with 80-100% inhibition, intermediate shRNA's with 50-80% inhibition and weak inhibitors with 0-50% inhibition. Of the 81 shRNA's tested, 21 were strong, 13 were intermediate and 47 were weak. To confirm the results from the 96-wells experiment, a selection was made of several shRNA's representing each group. These shRNA's were tested in a dilution series of 5, 25 and 100 ng respectively (figure 10). From the individual dose-response curves as well as from the average dose-response curves, it can be concluded that these shRNA's fall into the different functional groups, validating the data obtained from the screening of the 81 shRNA's.

### Table 1A Conserved regions in LAI

There are 19 different regions in the LAI genome that are highly conserved among different viral isolates. Shown here is the nucleotide position, genomic region and sequence of these different regions. ShRNA's were designed to target these regions, the numbers of the different shRNA expressing plasmids is shown as well as the name of the different shRNA constructs.

| start | | target sequence | number | name |
|---|---|---|---|---|
| 326 | leader | AAGGAGAGAGAUGGGUGCGAGAGCGUC | 1-9 | LDR 1-9 |
| 1146 | gag | AAUAAAAUAGUAAGAAUGUA | 10-11 | GAG 1-2 |
| 1363 | gag | UAGAAGAAAUGAUGACAGCAUG | 12-15 | GAG 3-6 |
| 1623 | gag/pol | CAGGCUAAUUUUUUAGGGAA | 16-17 | G/P 1-2 |
| 1910 | pol | ACAGGAGCAGAUGAUACAGU | 18-10 | POL 1-2 |
| 1957 | pol | AUGGAAACCAAAAAUGAUAGG | 20-22 | POL 3-6 |
| 3755 | pol | AUUGGAGGAAAUGAACAAGU | 23-24 | POL. 6-7 |
| 4121 | pol | UUAGCAGGAAGAUGGCCAGU | 25-26 | POL 8-9 |
| 4232 | pol | AUUCCCUACAAUCCCCAAAG | 27-28 | POL 10-11 |
| 4358 | pol | CACAAUUUUAAAAGAAAAGGGGGGAUUGGGGGG | 20-43 | POL 12-26 |
| 4391 | pol | UACAGUGCAGGGGAAAGAAUA | 44-46 | POL 27-29 |
| 4466 | pol | AAAAUUCAAAAUUUUCGGGU | 47-48 | POL 30-31 |
| 4470 | pol | UUCAAAAUUUUCGGGUUUAUU | 49-61 | POL 32-34 |
| 4475 | pol | AAUUUUCGGGUUUAUUACAG | 52-53 | POL 35-36 |
| 4535 | pol | CUCUGGAAAGGUGAAGGGGCAGUAGUAAU | 54-64 | POL 37-47 |
| 4622 | pol/vif | UAUGGAAAACAGAUGGCAGGUG | 85-88 | P/V 1-4 |
| 5547 | revitat | UCCUAUGGCAGGAAGAAGCGGAG | 69-73 | R/T 1-5 |
| 7391 | env | CAGCAGGAAGCACUAUGGGCGC | 74-77 | ENV 1-4 |
| 8656 | nef | GAAAAGGGGGGACUGGAAGGGCUAAUU | 78-86 | NEF 1-9 |

### Table 1B Conserved regions of HIV that are particularly suitable for targeting during HIV treatment

| Target sequence | Name |
|---|---|
| AGGAGAGAGAUGGGUGCGAGAGCGU | LDR 2-8 |
| UAGAAGAAAUGAUGACAGCAU | GAG 3-5 |
| ACAGGAGCAGAUGAUACAGU | POL 1-2 |
| AUUGGAGGAAAUGAACAAG | POL 6 |
| UAGCAGGAAGAUGGCCAGU | POL 9 |
| UACAGUGCAGGGGAAAGAA | POL 27 |
| CAGUGCAGGGGAAAGAAUA | POL 29 |
| AAGGUGAAGGGGCAGUAGUA | POL 44-45 |
| GUGAAGGGGCAGUAGUAAU | POL 47 |
| GGAAAACAGAUGGCAGGUG | P/V 4 |
| GGGGGGACUGGAAGGGCUAAUU | NEF 6-9 |

### Table 2A-2C shRNA's against conserved regions of HIV-1

Shown here is the percentage of inhibition for each shRNA and the sense sequence that is targeted by the shRNA. A division was made into three groups; strong, intermediate and weak shRNA's.

Table 2D The shRNA's targeting R/T1 to R/T5 which have not yet been tested.

**Table 2A Strong inhibition, 80%-100%**

| | | | |
|---|---|---|---|
| 3 | 99% | LDR3 | GGAGAGAGAUGGGUGCGAG |
| 5 | 98% | LDR5 | AGAGAGAUGGGUGCGAGAG |
| 2 | 97% | LDR2 | AGGAGAGAGAUGGGUGCGA |
| 18 | 97% | POL1 | ACAGGAGCAGAUGAUACAG |
| 4 | 96% | LDR4 | GAGAGAGAUGGGUGCGAGA |
| 64 | 96% | POL47 | GUGAAGGGGCAGUAGUAAU |
| 62 | 94% | POL45 | AGGUGAAGGGGCAGUAGUA |
| 19 | 92% | POL2 | CAGGAGCAGAUGAUACAGU |
| 9 | 91% | LDR9 | AGAUGGGUGCGAGAGCGUC |
| 6 | 91% | LDR6 | GAGAGAUGGGUGCGAGAGC |
| 8 | 91% | LDR8 | GAGAUGGGUGCGAGAGCGU |
| 7 | 91% | LDR7 | AGAGAUGGGUGCGAGAGCG |
| 86 | 90% | NEF9 | GGGACUGGAAGGGCUAAUU |
| 46 | 90% | POL29 | CAGUGCAGGGGAAAGAAUA |
| 13 | 89% | GAG4 | AGAAGAAAUGAUGACAGCA |
| 14 | 89% | GAG5 | GAAGAAAUGAUGACAGCAU |
| 85 | 86% | NEF8 | GGGGACUGGAAGGGCUAAU |
| 23 | 86% | POL6 | AUUGGAGGAAAUGAACAAG |
| 26 | 84% | POL9 | UAGCAGGAAGAUGGCCAGU |
| 68 | 83% | P/V4 | GGAAAACAGAUGGCAGGUG |
| 15 | 81% | GAG6 | AAGAAAUGAUGACAGCAUG |

**Table 2B Intermediate inhibition, 50%-80%**

| | | | |
|---|---|---|---|
| 61 | 79% | P0L44 | AAGGUGAAGGGGCAGUAGU |
| 44 | 79% | POL27 | UACAGUGCAGGGGAAAGAA |
| 84 | 76% | NEF7 | GGGGGACUGGAAGGGCUAA |
| 83 | 72% | NEF6 | GGGGGGACUGGAAGGGCUA |
| 25 | 70% | POL8 | UUAGCAGGAAGAUGGCCAG |
| 82 | 62% | NEF5 | AGGGGGGACUGGAAGGGCU |
| 35 | 59% | POL 18 | UUUAAAAGAAAAGGGGGGA |
| 58 | 58% | POL41 | GGAAAGGUGAAGGGGCAGU |
| 47 | 57% | POL3D | AAAAUUCAAAAUUUUCGGG |
| 59 | 56% | POL42 | GAAAGGUGAAGGGGCAGUA |
| 30 | 54% | POL13 | ACAAUUUUAAAAGAAAAGG |
| 27 | 53% | POL 10 | AUUCCCUACAAUCCCCAAA |
| 1 | 51% | LDR1 | AAGGAGAGAGAUGGGUGCG |

**Table 2C Weak inhibition 0%-50%**

| | | | |
|---|---|---|---|
| 81 | 44% | NEF4 | AAGGGGGGACUGGAAGGGC |
| 67 | 41% | P/V3 | UGGAAAACAGAUGGCAGGU |
| 12 | 40% | GAG3 | UAGAAGAAAUGAUGACAGC |
| 78 | 36% | NEF 1 | GAAAAGGGGGGACUGGAAG |
| 24 | 34% | POL7 | UUGGAGGAAAUGAACAAGU |
| 60 | 33% | POL43 | AAAGGUGAAGGGGCAGUAG |
| 42 | 33% | POL25 | GAAAAGGGGGGAUUGGGGG |
| 20 | 33% | POL3 | AUGGAAACCAAAAAUGAUA |
| 28 | 32% | POL11 | UUCCCUACAAUCCCCAAAG |
| 17 | 32% | G/P2 | AGGCUAAUUUUUUAGGGAA |
| 56 | 32% | POL39 | CUGGAAAGGUGAAGGGGCA |
| 21 | 30% | POL4 | UGGAAACCAAAAAUGAUAG |
| 80 | 29% | NEF3 | AAAGGGGGGACUGGAAGGG |
| 10 | 29% | GAG1 | AAUAAAAUAGUAAGAAUGU |
| 11 | 28% | GAG2 | AUAAAAUAGUAAGAAUGUA |
| 16 | 27% | G/P1 | CAGGCUAAUUUUUUAGGGA |
| 22 | 26% | POL5 | GGAAACCAAAAAUGAUAGG |
| 49 | 25% | POL32 | UUCAAAAUUUUCGGGUUUA |
| 52 | 25% | POL35 | AAUUUUCGGGUUUAUUACA |
| 51 | 24% | POL34 | CAAAAUUUUCGGGUUUAUU |
| 65 | 24% | P/V1 | UAUGGAAAACAGAUGGCAG |
| 66 | 24% | P/V2 | AUGGAAAACAGAUGGCAGG |
| 34 | 23% | POL17 | UUUUAAAAGAAAAGGGGGG |
| 55 | 20% | POL38 | UCUGGAAAGGUGAAGGGGC |
| 50 | 19% | POL33 | UCAAAAUUUUCGGGUUUAU |
| 45 | 19% | POL28 | ACAGUGCAGGGGAAAGAAU |
| 41 | 18% | POL24 | AGAAAAGGGGGGAUUGGGG |
| 38 | 17% | POL2 1 | AAAAGAAAAGGGGGGAUUG |
| 75 | 15% | ENV2 | AGCAGGAAGCACUAUGGGC |
| 39 | 14% | POL22 | AAAGAAAAGGGGGGAUUGG |
| 57 | 14% | POL40 | UGGAAAGGUGAAGGGGCAG |
| 79 | 14% | NEF2 | AAAAGGGGGGACUGGAAGG |
| 76 | 14% | ENV3 | GCAGGAAGCACUAUGGGCG |
| 63 | 13% | POL46 | GGUGAAGGGGCAGUAGUAA |
| 43 | 13% | POL26 | AAAAGGGGGGAUUGGGGGG |
| 54 | 12% | POL37 | CUCUGGAAAGGUGAAGGGG |
| 37 | 10% | POL20 | UAAAAGAAAAGGGGGGAUU |
| 29 | 8% | POL 12 | CACAAUUUUAAAAGAAAAG |
| 74 | 8% | ENV1 | CAGCAGGAAGCACUAUGGG |
| 53 | 6% | POL36 | AUUUUCGGGUUUAUUACAG |
| 31 | 5% | POL14 | CAAUUUUAAAAGAAAAGGG |
| 48 | 3% | POL31 | AAAUUCAAAAUUUUCGGGU |
| 33 | 2% | POL 16 | AUUUUAAAAGAAAAGGGGG |
| 32 | 0% | POL15 | AAUUUUAAAAGAAAAGGGG |
| 77 | 0% | ENV4 | CAGGAAGCACUAUGGGCGC |
| 40 | -2% | POL23 | AAGAAAAGGGGGGAUUGGG |
| 36 | -19% | POL19 | UUAAAAGAAAAGGGGGGAU |

**Table 2 D Not tested yet**

| | |
|---|---|
| R/T1 | UCCUAUGGCAGGAAGAAGC |
| R/T2 | CCUAUGGCAGGAAGAAGCG |
| R/T3 | CUAUGGCAGGAAGAAGCGG |
| R/T4 | UAUGGCAGGAAGAAGCGGA |
| R/T5 | AUGGCAGGAAGAAGCGGAG |

### Example 2

### Escape mutants

Besides targeting conserved regions, the number of escape routes is limited by selecting (conserved) regions of the viral genome that execute multiple functions. This includes overlapping reading frames, but also sequences that encode both a protein and an important RNA/DNA sequence (e.g. a splicing signal, or a sequence motif that controls RNA packaging or reverse transcription).

For example, the shRNA R/T5 targets the Tat1 reading frame and also the Rev1 reading frame. If we make the assumption that for both Tat and Rev, the amino acid sequence is conserved, then only mutations are allowed that are silent in both reading frames. For the shRNA targeting R/T5 this is shown in figure 8. Two silent mutations can occur in the shRNA R/T5 target: A6C or A18C (NB nt 1 is start of target sequence). Therefore, in addition to the primary shRNA, also shRNA's that are complementary to the two possible silent escape variants are preferably included in anti-HIV treatment.

Another example is P/V4 target, which was shown to be effectively targeted by a shRNA. The P/V4 target sequence contains the Pol and the Vif reading frames, figure 9. When we make again the assumption that only silent mutations are allowed, there is no escape possible for this target, no silent mutation can occur. Alternatively, based on protein structure, we predict what amino-acid substitutions for each reading frame are allowed. For these substitutions certain nucleic acid mutations are needed, again only the mutations that will result in both functional Pol and Vif proteins will be allowed, limiting the possibilities for escape. A second generation of shRNA's targeting these possible escape mutants are preferably combined with the primary shRNA.

For conserved regions of the HIV-1 genome that contain multiple regulatory RNA/DNA sequences and/or reading frames, similar exercises are performed. In each case, the possibilities of escape are limited due to the multiple functions of the target and are restricted to the few nucleotide positions for which variation is allowed. Only mutations are allowed that do not significantly impair any of its multiple functions. Primary shRNA's are preferably combined with the second generation to prevent escape.

Additionally, there is a preference for mismatches at certain positions. If we take a look at viral escape, and take into account the knowledge about the specificity of RNAi, it is striking that there is a preference in most virus escape mutants reported so far to have point mutations at the 9^{th} nucleotide position of the target mRNA. Three out of six reported escape mutants, that arose through a point mutation, have a point mutation at this position (Das et al., 2003, Boden et al., 2003 and Gitlin et al., 2002). Large scale testing of all the effective shRNA's we have found confirms this preference and/or shows that mutations at other positions result in effective evasion from RNAi. Combining the primary shRNA with a second generation anticipating this escape option(s) prevents escape.

Also, we have recently identified a novel resistance mechanism through the formation of stable RNA structure by (a) mutation(s) in or near the targeted sequence. This knowledge combined with computer-assisted RNA structure prediction, leads to a better design of a set of second generation shRNA's.

### Example 3

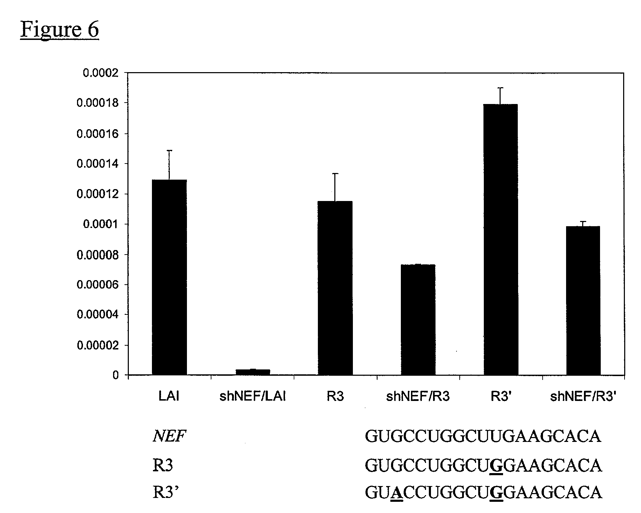

To test if the principle of anticipating escape is feasible, we tested this concept for two escape mutants against shNEF (Das et al., 2003). We designed complementary shRNA's targeting the escape mutants R3 and R3'. In a co-transfection experiment, LAI was strongly inhibited by the shNEF, while the molecular clones of the escape mutants R3 and R3' were almost insensitive (figure 6). The inhibition was about two-fold, which is executed at the level of translational silencing or at the level of RNAi. When we co-transfected these escape mutants with their complementary shRNA's, inhibition was restored to normal levels (figure 7). Therefore, as a proof of concept, we have shown that it is possible to counteract escape by designing a 2^{nd} generation shRNA complementary to an escape mutant.

### Figure legends

Figure **1**
   RNA interference with shRNA
Figure 2
   Anticipating and countering viral escape with a second generation shRNA
Figure 3
   RNAi induced by different RNA structures
Figure 4
   Regions in the HIV-1 RNA genome that are more than 75% conserved among different viral isolates
Figure 5
   shRNA's against conserved regions of HIV-1
   Shown here is the relative CA-p24 production, relative against CA-p24 of virus production in the absence of RNAi induced inhibition. CA-p24 was corrected for transfection efficiency by including Renilla in the transfection assay. shRNA expression plasmids (20 ng) and pLAI (100ng), a molecular clone of HIV-1, on 2 x 10e4 293T cells in a 96 wells format. About one in 4 shRNA's show a moderate to strong inhibition covering ten target regions.
Figure 6
   Escape mutants are much less inhibited by shNEF
   Shown here is the relative CA-p24 production, a measure for virus production corrected for transfection efficiency with renilla luciferase, after transfection of pLAI, the R3 or R3' escape mutant with or without the shNEF expression plasmid. The point mutations in the target sequences of R3 and R3' are bold and underlined. The escape mutants are much less inhibited by the shNEF. Since RNAi is highly sequence specific, the inhibition left is probably due to translational silencing, alternatively there could be some low level of RNAi still active.
Figure 7
   Proof of concept: 2^{nd} generation shRNA's can block escape mutants
   Shown here is the relative CA-p24 production, a measure for virus production corrected for transfection efficiency with renilla luciferase, after transfection of pLAI, the R3 or R3' escape mutant with or without the complementary shRNA: shNEF, shR3 or shR3' respectively. The point mutations in the target sequences of R3 and R3' are bold and underlined. Inhibition is restored to levels obtained with the shNEF and pLAI when complementary shRNA's are used against the escape mutants.
Figure 8
   The Rev1/Tat5 target of shRNA R/T5
   Shown here is the 19 nucleotide target of shRNA R/T4. For each reading frame the nucleotide sequence, codons and amino acid sequence is shown. There are two silent mutations possible for this target, a A 6 C or a A18C mutation.
Figure 9
   The Pol/Vif target of shRNA P/4
   Shown here is the 19 nucleotide target of shRNA P/4. For each reading frame the nucleotide sequence, codons and amino acid sequence is shown. There are no silent mutations possible for this target, only mutations that result in at least one amino-acid change of one of the reading frames.
Figure 10
   Validation of large shRNA screen
   A subset from the large screen was co-transfected in a dilution series with pLAI. The data are not corrected for transfection, shown are the CA-p24 data. As a positive control, the shRNA against the Nef gene (Das et al., 2003) was used, as a negative control a shRNA targeting the firefly luciferase gene or bluescript vector was used. The division in A, functional, B, intermediate and C, non-functional was based on the large screen, averages for each group are shown in D.

### References

1. Anderson et al, Oligonucleotides 13:303-312(2003)
2. Das et al. Journal of Virology, Vol 78, No. 5 (2004) pages 2601-2605
3. Gitlin et al. Nature, Vol 418 (2002) pages 430-434
4. Boden D, Pusch O, Lee F, Tucker L, Ramratnam B. J Virol. 2003 Nov;77(21):11531-5

### Annex to the application documents - subsequently filed sequences listing

SEQUENCE LISTING

<110>  Academisch Medisch Centrum bij de Universiteit van
       Amsterdam

<120>  Means and methods for counteracting an organism

<130>  P70419EP00

<140>  04077855.7
<141>  2004-10-15

<160>  128

<170>  PatentIn version 3.2

<210>  1
<211>  19
<212>  RNA
<213>  Artificial

<220>
<223>  sequence capable of inhibiting HIV escape mutant

<400>  1
gugccuggcu ggaagcaca                                                    19


<210>  2
<211>  19
<212>  RNA
<213>  Artificial

<220>
<223>  sequence capable of inhibiting HIV escape mutant

<400>  2
guaccuggcu ggaagcaca                                                    19


<210>  3
<211>  64
<212>  DNA
<213>  Artificial

<220>
<223>  oligo sequence wherein n is unknown


<220>
<221>  misc_feature
<222>  (8)..(26)
<223>  n is a, c, g, or t

<220>
<221>  misc_feature
<222>  (36)..(54)
<223>  n is a, c, g, or t

<400>  3
gatccccnnn nnnnnnnnnn nnnnnnttca agagannnnn nnnnnnnnnn nnnntttttg      60

gaaa                                                                    64

<210>  4
<211>  64
<212>  DNA
<213>  Artificial

<220>
<223>  oligo sequence wherein n is unknown

<220>
<221>  misc_feature
<222>  (15)..(33)
<223>  n is a, c, g, or t

<220>
<221>  misc_feature
<222>  (43)..(61)
<223>  n is a, c, g, or t

<400>  4
agcttttcca aaaannnnnn nnnnnnnnnn nnntctcttg aannnnnnnn nnnnnnnnnn      60

nggg                                                                    64

<210>  5
<211>  27
<212>  RNA
<213>  Human immunodeficiency virus

<220>
<221>  misc_feature
<222>  (1)..(27)

<400>  5
aaggagagag augggugcga gagcguc                                           27

<210>  6
<211>  20
<212>  RNA
<213>  Human immunodeficiency virus

<220>
<221>  misc_feature
<222>  (1)..(20)

<400>  6
aauaaaauag uaagaaugua                                                   20

<210>  7
<211>  22
<212>  RNA
<213>  Human immunodeficiency virus

<220>
<221>  misc_feature
<222>  (1)..(22)

<400>  7
uagaagaaau gaugacagca ug                                              22


<210>  8
<211>  20
<212>  RNA
<213>  Human immunodeficiency virus


<220>
<221>  misc_feature
<222>  (1)..(20)

<400>  8
caggcuaauu uuuuagggaa                                                 20


<210>  9
<211>  20
<212>  RNA
<213>  Human immunodeficiency virus


<220>
<221>  misc_feature
<222>  (1)..(20)

<400>  9
acaggagcag augauacagu                                                 20


<210>  10
<211>  21
<212>  RNA
<213>  Human immunodeficiency virus


<220>
<221>  misc_feature
<222>  (1)..(21)

<400>  10
auggaaacca aaaaugauag g                                               21


<210>  11
<211>  20
<212>  RNA
<213>  Human immunodeficiency virus


<220>
<221>  misc_feature
<222>  (1)..(20)

<400> 11
auuggaggaa augaacaagu 20

<210> 12
<211> 20
<212> RNA
<213> Human immunodeficiency virus

<220>
<221> misc_feature
<222> (1)..(20)

<400> 12
uuagcaggaa gauggccagu 20

<210> 13
<211> 20
<212> RNA
<213> Human immunodeficiency virus

<220>
<221> misc_feature
<222> (1)..(20)

<400> 13
auucccuaca auccccaaag 20

<210> 14
<211> 33
<212> RNA
<213> Human immunodeficiency virus

<220>
<221> misc_feature
<222> (1)..(33)

<400> 14
cacaauuuua aaagaaaagg ggggauuggg ggg 33

<210> 15
<211> 21
<212> RNA
<213> Human immunodeficiency virus

<220>
<221> misc_feature
<222> (1)..(21)

<400> 15
uacagugcag gggaaagaau a 21

<210>  16
<211>  20
<212>  RNA
<213>  Human immunodeficiency virus

<220>
<221>  misc_feature
<222>  (1)..(20)

<400>  16
aaaauucaaa auuuucgggu                                                    20

<210>  17
<211>  21
<212>  RNA
<213>  Human immunodeficiency virus

<220>
<221>  misc_feature
<222>  (1)..(21)

<400>  17
uucaaaauuu ucggguuuau u                                                  21

<210>  18
<211>  20
<212>  RNA
<213>  Human immunodeficiency virus

<220>
<221>  misc_feature
<222>  (1)..(20)

<400>  18
aauuuucggg uuuauuacag                                                    20

<210>  19
<211>  29
<212>  RNA
<213>  Human immunodeficiency virus

<220>
<221>  misc_feature
<222>  (1)..(29)

<400>  19
cucuggaaag gugaaggggc aguaguaau                                          29

<210>  20
<211>  22
<212>  RNA
<213>  Human immunodeficiency virus

<220>
<221>  misc_feature
<222>  (1)..(22)

<400>  20
uauggaaaac agauggcagg ug                                              22


<210>  21
<211>  23
<212>  RNA
<213>  Human immunodeficiency virus


<220>
<221>  misc_feature
<222>  (1)..(23)

<400>  21
uccuauggca ggaagaagcg gag                                             23


<210>  22
<211>  22
<212>  RNA
<213>  Human immunodeficiency virus


<220>
<221>  misc_feature
<222>  (1)..(22)

<400>  22
cagcaggaag cacuaugggc gc                                              22


<210>  23
<211>  27
<212>  RNA
<213>  Human immunodeficiency virus


<220>
<221>  misc_feature
<222>  (1)..(27)

<400>  23
gaaaaggggg gacuggaagg gcuaauu                                         27


<210>  24
<211>  25
<212>  RNA
<213>  Human immunodeficiency virus


<220>
<221>  misc_feature
<222>  (1)..(25)

<400>  24
aggagagaga ugggugcgag agcgu                                          25


<210>  25
<211>  21
<212>  RNA
<213>  Human immunodeficiency virus


<220>
<221>  misc_feature
<222>  (1)..(21)

<400>  25
uagaagaaau gaugacagca u                                              21


<210>  26
<211>  19
<212>  RNA
<213>  Human immunodeficiency virus


<220>
<221>  misc_feature
<222>  (1)..(19)

<400>  26
auuggaggaa augaacaag                                                 19


<210>  27
<211>  19
<212>  RNA
<213>  Human immunodeficiency virus


<220>
<221>  misc_feature
<222>  (1)..(19)

<400>  27
uagcaggaag auggccagu                                                 19


<210>  28
<211>  19
<212>  RNA
<213>  Human immunodeficiency virus


<220>
<221>  misc_feature
<222>  (1)..(19)

<400>  28
uacagugcag gggaaagaa                                                 19


<210>  29

<211>  19
<212>  RNA
<213>  Human immunodeficiency virus

<220>
<221>  misc_feature
<222>  (1)..(19)

<400>  29
cagugcaggg gaaagaaua                                                    19

<210>  30
<211>  20
<212>  RNA
<213>  Human immunodeficiency virus

<220>
<221>  misc_feature
<222>  (1)..(20)

<400>  30
aaggugaagg ggcaguagua                                                   20

<210>  31
<211>  19
<212>  RNA
<213>  Human immunodeficiency virus

<220>
<221>  misc_feature
<222>  (1)..(19)

<400>  31
gugaaggggc aguaguaau                                                    19

<210>  32
<211>  19
<212>  RNA
<213>  Human immunodeficiency virus

<220>
<221>  misc_feature
<222>  (1)..(19)

<400>  32
ggaaaacaga uggcaggug                                                    19

<210>  33
<211>  22
<212>  RNA
<213>  Human immunodeficiency virus

<220>
<221>  misc_feature
<222>  (1)..(22)

<400>  33
gggggacug gaagggcuaa uu                                                22


<210>  34
<211>  19
<212>  RNA
<213>  Human immunodeficiency virus


<220>
<221>  misc_feature
<222>  (1)..(19)

<400>  34
ggagagagau gggugcgag                                                   19


<210>  35
<211>  19
<212>  RNA
<213>  Human immunodeficiency virus


<220>
<221>  misc_feature
<222>  (1)..(19)

<400>  35
agagagaugg gugcgagag                                                   19


<210>  36
<211>  19
<212>  RNA
<213>  Human immunodeficiency virus


<220>
<221>  misc_feature
<222>  (1)..(19)

<400>  36
aggagagaga ugggugcga                                                   19


<210>  37
<211>  19
<212>  RNA
<213>  Human immunodeficiency virus


<220>
<221>  misc_feature
<222>  (1)..(19)

<400>  37

acaggagcag augauacag                                                    19

<210>  38
<211>  19
<212>  RNA
<213>  Human immunodeficiency virus

<220>
<221>  misc_feature
<222>  (1)..(19)

<400>  38
gagagagaug ggugcgaga                                                    19

<210>  39
<211>  19
<212>  RNA
<213>  Human immunodeficiency virus

<220>
<221>  misc_feature
<222>  (1)..(19)

<400>  39
caggagcaga ugauacagu                                                    19

<210>  40
<211>  19
<212>  RNA
<213>  Human immunodeficiency virus

<220>
<221>  misc_feature
<222>  (1)..(19)

<400>  40
agaugggugc gagagcguc                                                    19

<210>  41
<211>  19
<212>  RNA
<213>  Human immunodeficiency virus

<220>
<221>  misc_feature
<222>  (1)..(19)

<400>  41
gagagauggg ugcgagagc                                                    19

<210>  42
<211>  19

<212>  RNA
<213>  Human immunodeficiency virus

<220>
<221>  misc_feature
<222>  (1)..(19)

<400>  42
gagaugggug cgagagcgu                                                    19

<210>  43
<211>  19
<212>  RNA
<213>  Human immunodeficiency virus

<220>
<221>  misc_feature
<222>  (1)..(19)

<400>  43
agagaugggu gcgagagcg                                                    19

<210>  44
<211>  19
<212>  RNA
<213>  Human immunodeficiency virus

<220>
<221>  misc_feature
<222>  (1)..(19)

<400>  44
gggacuggaa gggcuaauu                                                    19

<210>  45
<211>  19
<212>  RNA
<213>  Human immunodeficiency virus

<220>
<221>  misc_feature
<222>  (1)..(19)

<400>  45
agaagaaaug augacagca                                                    19

<210>  46
<211>  19
<212>  RNA
<213>  Human immunodeficiency virus

<220>

<221>  misc_feature
<222>  (1)..(19)

<400>  46
gaagaaauga ugacagcau                                                    19


<210>  47
<211>  19
<212>  RNA
<213>  Human immunodeficiency virus


<220>
<221>  misc_feature
<222>  (1)..(19)

<400>  47
ggggacugga agggcuaau                                                    19


<210>  48
<211>  19
<212>  RNA
<213>  Human immunodeficiency virus


<220>
<221>  misc_feature
<222>  (1)..(19)

<400>  48
aagaaaugau gacagcaug                                                    19


<210>  49
<211>  19
<212>  RNA
<213>  Human immunodeficiency virus


<220>
<221>  misc_feature
<222>  (1)..(19)

<400>  49
aaggugaagg ggcaguagu                                                    19


<210>  50
<211>  19
<212>  RNA
<213>  Human immunodeficiency virus


<220>
<221>  misc_feature
<222>  (1)..(19)

<400>  50
uacagugcag gggaaagaa                                                    19

<210>  51
<211>  19
<212>  RNA
<213>  Human immunodeficiency virus

<220>
<221>  misc_feature
<222>  (1)..(19)

<400>  51
gggggacugg aagggcuaa                                                    19

<210>  52
<211>  19
<212>  RNA
<213>  Human immunodeficiency virus

<220>
<221>  misc_feature
<222>  (1)..(19)

<400>  52
ggggggacug gaagggcua                                                    19

<210>  53
<211>  19
<212>  RNA
<213>  Human immunodeficiency virus

<220>
<221>  misc_feature
<222>  (1)..(19)

<400>  53
uuagcaggaa gauggccag                                                    19

<210>  54
<211>  19
<212>  RNA
<213>  Human immunodeficiency virus

<220>
<221>  misc_feature
<222>  (1)..(19)

<400>  54
aggggggacu ggaagggcu                                                    19

<210>  55
<211>  19
<212>  RNA

<213>  Human immunodeficiency virus

<220>
<221>  misc_feature
<222>  (1)..(19)

<400>  55
   uuuaaaagaa aagggggga                                                    19

<210>  56
<211>  19
<212>  RNA
<213>  Human immunodeficiency virus

<220>
<221>  misc_feature
<222>  (1)..(19)

<400>  56
ggaaagguga aggggcagu                                                   19

<210>  57
<211>  19
<212>  RNA
<213>  Human immunodeficiency virus

<220>
<221>  misc_feature
<222>  (1)..(19)

<400>  57
aaaauucaaa auuuucggg                                                   19

<210>  58
<211>  19
<212>  RNA
<213>  Human immunodeficiency virus

<220>
<221>  misc_feature
<222>  (1)..(19)

<400>  58
gaaaggugaa ggggcagua                                                   19

<210>  59
<211>  19
<212>  RNA
<213>  Human immunodeficiency virus

<220>
<221>  misc_feature

<222>  (1)..(19)

<400>  59
acaauuuuaa aagaaaagg                                                    19


<210>  60
<211>  19
<212>  RNA
<213>  Human immunodeficiency virus


<220>
<221>  misc_feature
<222>  (1)..(19)

<400>  60
auucccuaca auccccaaa                                                    19


<210>  61
<211>  19
<212>  RNA
<213>  Human immunodeficiency virus


<220>
<221>  misc_feature
<222>  (1)..(19)

<400>  61
aaggagagag augggugcg                                                    19


<210>  62
<211>  19
<212>  RNA
<213>  Human immunodeficiency virus


<220>
<221>  misc_feature
<222>  (1)..(19)

<400>  62
   aaggggggac uggaagggc                                                     19


<210>  63
<211>  19
<212>  RNA
<213>  Human immunodeficiency virus


<220>
<221>  misc_feature
<222>  (1)..(19)

<400>  63
uggaaaacag auggcaggu                                                    19

<210>  64
<211>  19
<212>  RNA
<213>  Human immunodeficiency virus


<220>
<221>  misc_feature
<222>  (1)..(19)

<400>  64
uagaagaaau gaugacagc                                                    19


<210>  65
<211>  19
<212>  RNA
<213>  Human immunodeficiency virus


<220>
<221>  misc_feature
<222>  (1)..(19)

<400>  65
gaaaaggggg gacuggaag                                                    19


<210>  66
<211>  19
<212>  RNA
<213>  Human immunodeficiency virus


<220>
<221>  misc_feature
<222>  (1)..(19)

<400>  66
uuggaggaaa ugaacaagu                                                    19


<210>  67
<211>  19
<212>  RNA
<213>  Human immunodeficiency virus


<220>
<221>  misc_feature
<222>  (1)..(19)

<400>  67
aaaggugaag gggcaguag                                                    19


<210>  68
<211>  19
<212>  RNA
<213>  Human immunodeficiency virus

<220>
<221>  misc_feature
<222>  (1)..(19)

<400>  68
gaaaaggggg gauuggggg                                                    19


<210>  69
<211>  19
<212>  RNA
<213>  Human immunodeficiency virus


<220>
<221>  misc_feature
<222>  (1)..(19)

<400>  69
uaggaaacca aaaaugaua                                                    19


<210>  70
<211>  19
<212>  RNA
<213>  Human immunodeficiency virus


<220>
<221>  misc_feature
<222>  (1)..(19)

<400>  70
uucccuacaa uccccaaag                                                    19


<210>  71
<211>  19
<212>  RNA
<213>  Human immunodeficiency virus


<220>
<221>  misc_feature
<222>  (1)..(19)

<400>  71
aggcuaauuu uuuagggaa                                                    19


<210>  72
<211>  19
<212>  RNA
<213>  Human immunodeficiency virus


<220>
<221>  misc_feature
<222>  (1)..(19)

<400>  72
cuggaaaggu gaaggggca                                                    19


<210>  73
<211>  19
<212>  RNA
<213>  Human immunodeficiency virus


<220>
<221>  misc_feature
<222>  (1)..(19)

<400>  73
uggaaaccaa aaaugauag                                                    19


<210>  74
<211>  19
<212>  RNA
<213>  Human immunodeficiency virus


<220>
<221>  misc_feature
<222>  (1)..(19)

<400>  74
aaagggggga cuggaaggg                                                    19


<210>  75
<211>  19
<212>  RNA
<213>  Human immunodeficiency virus


<220>
<221>  misc_feature
<222>  (1)..(19)

<400>  75
aauaaaauag uaagaaugu                                                    19


<210>  76
<211>  19
<212>  RNA
<213>  Human immunodeficiency virus


<220>
<221>  misc_feature
<222>  (1)..(19)

<400>  76
auaaaauagu aagaaugua                                                    19

<210>  77
<211>  19
<212>  RNA
<213>  Human immunodeficiency virus

<220>
<221>  misc_feature
<222>  (1)..(19)

<400>  77
caggcuaauu uuuuaggga                                                    19

<210>  78
<211>  19
<212>  RNA
<213>  Human immunodeficiency virus

<220>
<221>  misc_feature
<222>  (1)..(19)

<400>  78
ggaaaccaaa aaugauagg                                                    19

<210>  79
<211>  19
<212>  RNA
<213>  Human immunodeficiency virus

<220>
<221>  misc_feature
<222>  (1)..(19)

<400>  79
uucaaaauuu ucggguuua                                                    19

<210>  80
<211>  19
<212>  RNA
<213>  Human immunodeficiency virus

<220>
<221>  misc_feature
<222>  (1)..(19)

<400>  80
aauuuucggg uuuauuaca                                                    19

<210>  81
<211>  19
<212>  RNA
<213>  Human immunodeficiency virus

<220>
<221>  misc_feature
<222>  (1)..(19)

<400>  81
caaaauuuc ggguuuauu                                                    19


<210>  82
<211>  19
<212>  RNA
<213>  Human immunodeficiency virus


<220>
<221>  misc_feature
<222>  (1)..(19)

<400>  82
   uauggaaaac agauggcag                                                    19


<210>  83
<211>  19
<212>  RNA
<213>  Human immunodeficiency virus


<220>
<221>  misc_feature
<222>  (1)..(19)

<400>  83
   auggaaaaca gauggcagg                                                    19


<210>  84
<211>  19
<212>  RNA
<213>  Human immunodeficiency virus


<220>
<221>  misc_feature
<222>  (1)..(19)

<400>  84
   uuuuaaaaga aaagggggg                                                    19


<210>  85
<211>  19
<212>  RNA
<213>  Human immunodeficiency virus


<220>
<221>  misc_feature
<222>  (1)..(19)

<400>  85
ucuggaaagg ugaaggggc                                                    19


<210>  86
<211>  19
<212>  RNA
<213>  Human immunodeficiency virus


<220>
<221>  misc_feature
<222>  (1)..(19)

<400>  86
ucaaaauuuu cggguuuau                                                    19


<210>  87
<211>  19
<212>  RNA
<213>  Human immunodeficiency virus


<220>
<221>  misc_feature
<222>  (1)..(19)

<400>  87
acagugcagg ggaaagaau                                                    19


<210>  88
<211>  19
<212>  RNA
<213>  Human immunodeficiency virus


<220>
<221>  misc_feature
<222>  (1)..(19)

<400>  88
agaaaagggg ggauugggg                                                    19


<210>  89
<211>  19
<212>  RNA
<213>  Human immunodeficiency virus


<220>
<221>  misc_feature
<222>  (1)..(19)

<400>  89
aaaagaaaag gggggauug                                                    19


<210>  90

<211>  19
<212>  RNA
<213>  Human immunodeficiency virus


<220>
<221>  misc_feature
<222>  (1)..(19)

<400>  90
agcaggaagc acuaugggc                                                    19


<210>  91
<211>  19
<212>  RNA
<213>  Human immunodeficiency virus


<220>
<221>  misc_feature
<222>  (1)..(19)

<400>  91
aaagaaaagg ggggauugg                                                    19


<210>  92
<211>  19
<212>  RNA
<213>  Human immunodeficiency virus


<220>
<221>  misc_feature
<222>  (1)..(19)

<400>  92
uggaaaggug aaggggcag                                                    19


<210>  93
<211>  19
<212>  RNA
<213>  Human immunodeficiency virus


<220>
<221>  misc_feature
<222>  (1)..(19)

<400>  93
aaaagggggg acuggaagg                                                    19


<210>  94
<211>  19
<212>  RNA
<213>  Human immunodeficiency virus

<220>
<221> misc_feature
<222> (1)..(19)

<400> 94
gcaggaagca cuaugggcg 19


<210> 95
<211> 19
<212> RNA
<213> Human immunodeficiency virus


<220>
<221> misc_feature
<222> (1)..(19)

<400> 95
ggugaagggg caguaguaa 19


<210> 96
<211> 19
<212> RNA
<213> Human immunodeficiency virus


<220>
<221> misc_feature
<222> (1)..(19)

<400> 96
   aaaagggggg auugggggg 19


<210> 97
<211> 19
<212> RNA
<213> Human immunodeficiency virus


<220>
<221> misc_feature
<222> (1)..(19)

<400> 97
cucuggaaag gugaagggg 19


<210> 98
<211> 19
<212> RNA
<213> Human immunodeficiency virus


<220>
<221> misc_feature
<222> (1)..(19)

<400> 98

uaaaagaaaa gggggauu                                                    19

<210>  99
<211>  19
<212>  RNA
<213>  Human immunodeficiency virus

<220>
<221>  misc_feature
<222>  (1)..(19)

<400>  99
cacaauuuua aaagaaaag                                                   19

<210>  100
<211>  19
<212>  RNA
<213>  Human immunodeficiency virus

<220>
<221>  misc_feature
<222>  (1)..(19)

<400>  100
cagcaggaag cacuauggg                                                   19

<210>  101
<211>  19
<212>  RNA
<213>  Human immunodeficiency virus

<220>
<221>  misc_feature
<222>  (1)..(19)

<400>  101
auuuucgggu uuauuacag                                                   19

<210>  102
<211>  19
<212>  RNA
<213>  Human immunodeficiency virus

<220>
<221>  misc_feature
<222>  (1)..(19)

<400>  102
caauuuuaaa agaaaaggg                                                   19

<210>  103
<211>  19

<212>  RNA
<213>  Human immunodeficiency virus

<220>
<221>  misc_feature
<222>  (1)..(19)

<400>  103
   aaauucaaaa uuuucgggu                                                    19

<210>  104
<211>  19
<212>  RNA
<213>  Human immunodeficiency virus

<220>
<221>  misc_feature
<222>  (1)..(19)

<400>  104
   auuuuaaaag aaaaggggg                                                    19

<210>  105
<211>  19
<212>  RNA
<213>  Human immunodeficiency virus

<220>
<221>  misc_feature
<222>  (1)..(19)

<400>  105
   aauuuuaaaa gaaaagggg                                                    19

<210>  106
<211>  19
<212>  RNA
<213>  Human immunodeficiency virus

<220>
<221>  misc_feature
<222>  (1)..(19)

<400>  106
caggaagcac uaugggcgc                                                   19

<210>  107
<211>  19
<212>  RNA
<213>  Human immunodeficiency virus

<220>

<221>  misc_feature
<222>  (1)..(19)

<400>  107
aagaaaaggg gggauuggg                                                    19


<210>  108
<211>  19
<212>  RNA
<213>  Human immunodeficiency virus


<220>
<221>  misc_feature
<222>  (1)..(19)

<400>  108
uuaaaagaaa aggggggau                                                    19


<210>  109
<211>  19
<212>  RNA
<213>  Human immunodeficiency virus


<220>
<221>  misc_feature
<222>  (1)..(19)

<400>  109
uccuauggca ggaagaagc                                                    19


<210>  110
<211>  19
<212>  RNA
<213>  Human immunodeficiency virus


<220>
<221>  misc_feature
<222>  (1)..(19)

<400>  110
ccuauggcag gaagaagcg                                                    19


<210>  111
<211>  19
<212>  RNA
<213>  Human immunodeficiency virus


<220>
<221>  misc_feature
<222>  (1)..(19)

<400>  111
cuauggcagg aagaagcgg                                                    19

<210>  112
<211>  19
<212>  RNA
<213>  Human immunodeficiency virus

<220>
<221>  misc_feature
<222>  (1)..(19)

<400>  112
uauggcagga agaagcgga                                                    19

<210>  113
<211>  19
<212>  RNA
<213>  Human immunodeficiency virus

<220>
<221>  misc_feature
<222>  (1)..(19)

<400>  113
auggcaggaa gaagcggag                                                    19

<210>  114
<211>  49
<212>  RNA
<213>  Artificial

<220>
<223>  sh RNA

<400>  114
gugccuggcu agaagcacau ucaagagaug ugcuucuagc caggcacuu                   49

<210>  115
<211>  21
<212>  RNA
<213>  Artificial

<220>
<223>  sense strand of siRNA

<400>  115
gugccuggcu agaagcacau u                                                 21

<210>  116
<211>  21
<212>  RNA
<213>  Artificial

<220>
<223>  antisense strand of siRNA

<400>  116
ugugcuucua gccaggcacu u                                                21


<210>  117
<211>  57
<212>  RNA
<213>  Artificial

<220>
<223>  target mRNA

<400>  117
agcagcuacc aaugcugcuu gugccuggcu agaagcacaa gaggaggagg agguggg          57


<210>  118
<211>  57
<212>  RNA
<213>  Human immunodeficiency virus


<220>
<221>  misc_feature
<222>  (1)..(57)

<400>  118
agcagcuacc aaugcugcuu guaccuggcu ggaagcacaa gaggaggagg agguggg          57


<210>  119
<211>  21
<212>  RNA
<213>  Artificial

<220>
<223>  2nd generation si/sh RNA

<400>  119
ugugcuucca gccagguacu u                                                21


<210>  120
<211>  23
<212>  RNA
<213>  Human immunodeficiency virus


<220>
<221>  misc_feature
<222>  (1)..(23)

<400>  120
gcauccagga agucagccua aaa                                              23


<210>  121
<211>  47
<212>  RNA
<213>  Artificial

<220>
<223>  sh RNA wherein n is unknown

<220>
<221>  misc_feature
<222>  (1)..(18)
<223>  n is a, c, g, or u

<220>
<221>  misc_feature
<222>  (28)..(45)
<223>  n is a, c, g, or u

<400>  121
nnnnnnnnnn nnnnnnnnuu caagagannn nnnnnnnnnn nnnnnuu                47

<210>  122
<211>  19
<212>  RNA
<213>  Human immunodeficiency virus

<220>
<221>  misc_feature
<222>  (1)..(19)

<400>  122
gugccuggcu ugaagcaca                                               19

<210>  123
<211>  8
<212>  PRT
<213>  Human immunodeficiency virus

<220>
<221>  SITE
<222>  (1)..(8)

<400>  123

Ser Tyr Gly Arg Lys Lys Arg Arg
1               5

<210>  124
<211>  50
<212>  DNA
<213>  Human immunodeficiency virus

<220>
<221>  misc_feature
<222>  (1)..(50)

<400>  124
tcctatggca ggaagaagcg gagactccta tggcaggaag aagcggagac             50

<210>  125
<211>  7
<212>  PRT
<213>  Human immunodeficiency virus


<220>
<221>  SITE
<222>  (1)..(7)

<400>  125

Met Ala Gly Arg Ser Gly Asp
1               5


<210>  126
<211>  7
<212>  PRT
<213>  Human immunodeficiency virus


<220>
<221>  SITE
<222>  (1)..(7)

<400>  126

Gly Lys Gln Met Ala Gly Asp
1               5


<210>  127
<211>  52
<212>  DNA
<213>  Human immunodeficiency virus


<220>
<221>  misc_feature
<222>  (1)..(52)

<400>  127
attatggaaa acagatggca ggtgatatta tggaaaacag atggcaggtg at          52


<210>  128
<211>  7
<212>  PRT
<213>  Human immunodeficiency virus


<220>
<221>  SITE
<222>  (1)..(7)

<400>  128

Met Glu Asn Arg Trp Gln Val

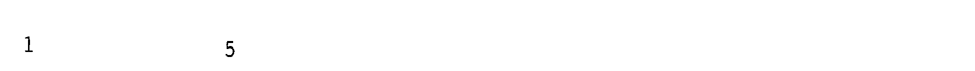

## Claims

1. A nucleic acid molecule comprising a sequence which is complementary to a mutant of a genomic nucleic acid sequence of an organism capable of mutating its genome.

2. A nucleic acid molecule according to claim 1, wherein said organism comprises a virus.

3. A nucleic acid molecule according to claim 1 or 2, wherein said organism comprises HIV, Hepatitis B, Hepatitis C, SARS, Ebola, Influenza, West Nile and/or polio virus.

4. A nucleic acid molecule according to any one of claims 1-3, wherein said genomic nucleic acid sequence of said organism comprises at least part of an essential region.

5. A nucleic acid molecule according to any one of claims 1-4, wherein said genomic nucleic acid sequence of said organism comprises at least part of a conserved region.

6. A nucleic acid molecule according to any one of claims 1-5, wherein said conserved region comprises a region as depicted in table 1B.

7. A nucleic acid molecule according to any one of claims 1-6, wherein said genomic nucleic acid sequence of said organism comprises at least one coding sequence and/or at least one DNA/RNA regulatory sequence.

8. A nucleic acid molecule according to any one of claims 1-7, wherein said mutant comprises a coding sequence of said organism wherein a nucleotide of a codon is substituted such that the resulting codon encodes the same amino acid residue.

9. A nucleic acid molecule according to any one of claims 1-8, wherein said mutant comprises a genomic nucleic acid sequence of said organism with at least one mutation resulting in a significantly altered structure of said genomic nucleic acid sequence.

10. A nucleic acid molecule according to any one of claims 1-9, comprising small interfering RNA.

11. A nucleic acid molecule according to any one of claims 1-10, comprising short hairpin RNA.

12. A nucleic acid molecule according to any one of claims 1-11, comprising an inducible repressor and/or activator.

13. A nucleic acid molecule according to any one of claims 1-12, comprising a sequence which is complementary to a mutant of a sequence as depicted in table 2A, 2B or 2C, or a functional part, derivative and/or analogue thereof.

14. A nucleic acid molecule according to any one of claims 1-13, comprising a sequence which is complementary to a mutant of a sequence as depicted in table 2A or 2B, or a functional part, derivative and/or analogue thereof.

15. A nucleic acid molecule according to any one of claims 1-14, comprising a sequence which is complementary to a mutant of a sequence as depicted in table 2A, or a functional part, derivative and/or analogue thereof.

16. A nucleic acid molecule according to any one of claims 1-15, which is complementary to the sequence GUGCCUGGCUGGAAGCACA and/or GUACCUGGCUGGAAGCACA, or a functional part, derivative and/or analogue thereof.

17. A nucleic acid molecule comprising a sequence which is complementary to a conserved region as depicted in table 1B, or a functional part, derivative and/or analogue thereof.

18. A nucleic acid according to claim 17, comprising a sequence which is complementary to a sequence as depicted in table 2A or 2B, or a functional part, derivative and/or analogue thereof.

19. A nucleic acid according to claim 17 or 18, comprising a sequence which is complementary to a sequence as depicted in table 2A, or a functional part, derivative and/or analogue thereof.

20. A nucleic acid molecule according to any one of claims 1-19 for use as a medicament.

21. Use of a nucleic acid molecule according to any one of claims 1-19 for the preparation of a medicament against a disease involving an organism capable of mutating its genome.

22. Use of a nucleic acid molecule according to any one of claims 1-19 for the preparation of a medicament against HIV, Hepatitis B, Hepatitis C, SARS, Ebola, Influenza, West Nile and/or polio virus.

23. A pharmaceutical composition comprising a nucleic acid molecule according to any one of claims 1-19 and a suitable diluent or carrier.

24. A pharmaceutical composition according to claim 23, further comprising a nucleic acid molecule comprising a sequence which is complementary to said genomic nucleic acid sequence of said organism.

25. A pharmaceutical composition according to claim 23 or 24, further comprising another kind of drug capable of at least in part counteracting said disease.

26. A pharmaceutical composition according to any one of claims 23-25, comprising nucleic acid sequences which are complementary to mutants of at least two genomic nucleic acid sequences of said organism.

27. A pharmaceutical composition according to any one of claims 23-26, comprising nucleic acid sequences which are complementary to mutants of at least six genomic nucleic acid sequences of said organism.

28. A gene delivery vehicle comprising a nucleic acid sequence according to any one of claims 1-19.

29. A method for at least in part counteracting an organism, comprising providing said organism with a nucleic acid molecule according to any one of claims 1-19.

30. A method according to claim 29, wherein said organism is additionally provided with a nucleic acid molecule comprising a sequence which is complementary to said genomic nucleic acid sequence of said organism.

31. A method according to claims 29 or 30, wherein said organism is provided with nucleic acid sequences which are complementary to mutants of at least two genomic nucleic acid sequences of said organism.

32. A method according to any one of claims 29-31, wherein said organism is provided with nucleic acid sequences which are complementary to mutants of at least six genomic nucleic acid sequences of said organism.

33. A method for interfering with a cell comprising an organism capable of mutating its genome and/or a nucleic acid sequence of an organism capable of mutating its genome, comprising providing said cell with a nucleic acid sequence according to any one of claims 1-19.

34. A method for at least in part treating or preventing a disease involved with an organism capable of mutating its genome, comprising providing an individual suffering from, or at risk of suffering from, said disease with a pharmaceutical composition according to any one of claims 23-27 or a gene delivery vehicle according to claim 28.

35. A method according to claim 33 or 34, further comprising providing said cell or said individual with another kind of drug capable of at least in part counteracting said organism.

36. A kit of parts comprising a nucleic acid molecule according to any one of claims 1-19 and another kind of drug capable of at least in part counteracting said organism.
